# EUROPEAN PATENT APPLICATION

(11) **EP 3 949 986 A1**
(43) Date of publication of application: **09.02.2022**
(21) Application number: 20777049.6
(22) Date of filing: 26.03.2020
(51) Int. Cl.: A61K 39/395, A61K 45/00, A61P 1/16, A61P 31/20, A61P 43/00, C12N 15/09, C12N 15/113, C12N 15/864, C12Q 1/686, A61K 38/02, G01N 33/15, G01N 33/50, A61K 31/7088, A61K 31/713, A61K 35/76

(54) **PHARMACEUTICAL COMPOSITION FOR INHIBITING PRODUCTION OF HEPATITIS B VIRUS PROTEIN, AND METHOD FOR SCREENING SAME**

(30) Priority: 26.03.2019 JP 2019058788
(71) Applicant: FUJIFILM Corporation, Tokyo 106-8620 (JP); Public University Corporation Nagoya City University, Mizuho-ku Nagoya-shi Aichi 467-8601 (JP)
(72) Inventor: TANAKA Yasuhito, Nagoya-shi, Aichi 467-8601 (JP); HABA Ryota, Toyama-shi, Toyama 930-8508 (JP); SHIMANE Kazuki, Toyama-shi, Toyama 930-8508 (JP); KAWANO Takaki, Toyama-shi, Toyama 930-8508 (JP)
(74) Representative: Hoffmann Eitle
(86) International application number: PCT/JP2020/013624
(87) International publication number: WO 2020/196737

(57) **Abstract**

An object of the present invention is to provide a pharmaceutical composition useful as a novel anti-hepatitis B virus agent and a screening method. According to the present invention, there is provided a pharmaceutical composition that inhibits a production of a hepatitis B virus protein, in which the pharmaceutical composition inhibits an expression or a function of an RNA-binding protein that binds to at least one sequence selected from a hepatitis B virus RNA sequence corresponding to an enhancer I region sequence on a hepatitis B virus genome DNA or a hepatitis B virus RNA sequence corresponding to an enhancer II region sequence on the hepatitis B virus genome DNA.

### [Sequence listing] 19F00712W1JP20013624_6.app

SEQUENCE LISTING
<110>  FUJIFILM Corporation
<120>  Pharmaceutical composition which inhibits production of Hepatitis B
virus protein and screening method
<130>  19F00712W1
<160>  31
<170>  PatentIn version 3.5
<210>  1
<211>  201
<212>  RNA
<213>  Hepatitis B virus
<400>  1
uuauaugcau guauacaauc uaagcaggcu uucacuuucu cgccaacuua caaggccuuu      60
cuguguaaac aauaucugaa ccuuuacccc guugcccggc aacggucagg ucucugccaa     120
guguuugcug acgcaacccc cacuggaugg ggcuuggcua ucggccaucg ccgcaugcgu     180
ggaaccuuug uggcuccgcu g                                               201
<210>  2
<211>  134
<212>  RNA
<213>  Hepatitis B virus
<400>  2
uugcccaagg ucuuauauaa gaggacucuu ggacucucag cgaugucaac gaccgaccuu      60
gaggcauacu ucaaagacug uuuguuuaag gacugggagg aguuggggga ggagauuagg     120
uuaaagauuu uugu                                                       134
<210>  3
<211>  30
<212>  DNA
<213>  Artificial Sequence
<220>
<223>  Description of Artificial Sequence: primer
<400>  3
gtcaggtacc caaacaatcc agattgggac                                       30
<210>  4
<211>  26
<212>  DNA
<213>  Artificial Sequence
<220>
<223>  Description of Artificial Sequence: primer
<400>  4
tgacaagctt gttcggtgca gggtcc                                           26
<210>  5
<211>  30
<212>  DNA
<213>  Artificial Sequence
<220>
<223>  Description of Artificial Sequence: primer
<400>  5
tgactctaga accctaataa aaccaaacgt                                       30
<210>  6
<211>  30
<212>  DNA
<213>  Artificial Sequence
<220>
<223>  Description of Artificial Sequence: primer
<400>  6
tgacgtcgac tttatacggg tcaatgtcca                                       30
<210>  7
<211>  20
<212>  DNA
<213>  Artificial Sequence
<220>
<223>  Description of Artificial Sequence: primer
<400>  7

cgcggggcat gactatcgtc                                                    20
<210>  8
<211>  20
<212>  DNA
<213>  Artificial Sequence
<220>
<223>  Description of Artificial Sequence: primer
<400>  8
aggcattaag gcaggatagc                                                    20
<210>  9
<211>  30
<212>  DNA
<213>  Artificial Sequence
<220>
<223>  Description of Artificial Sequence: primer
<400>  9
cctgccttaa tgcctccgat ccatactgcg                                         30
<210>  10
<211>  20
<212>  DNA
<213>  Artificial Sequence
<220>
<223>  Description of Artificial Sequence: primer
<400>  10
gacgatagtc atgccccgcg                                                    20
<210>  11
<211>  20
<212>  DNA
<213>  Artificial Sequence
<220>
<223>  Description of Artificial Sequence: primer
<400>  11
gacctggtgg gcgttcacgg                                                    20
<210>  12
<211>  30
<212>  DNA
<213>  Artificial Sequence
<220>
<223>  Description of Artificial Sequence: primer
<400>  12
aacgcccacc aggtcactag gaggctgtag                                         30

<210>  13
<211>  30
<212>  DNA
<213>  Artificial Sequence
<220>
<223>  Description of Artificial Sequence: primer
<400>  1
tagttctaga accctaacaa aacaaaaaga                                         30
<210>  14
<211>  30
<212>  DNA
<213>  Artificial Sequence
<220>
<223>  Description of Artificial Sequence: primer
<400>  14
tagtgtcgac tttataaggg tcaatgtcca                                         30
<210>  15
<211>  30
<212>  DNA
<213>  Artificial Sequence
<220>

<223>  Description of Artificial Sequence: primer
<400>  15
cctgccttaa tgcctccgat ccatactgcg                                    30
<210>  16
<211>  20
<212>  DNA
<213>  Artificial Sequence
<220>
<223>  Description of Artificial Sequence: primer
<400>  16
aggcattaag gcaggatatc                                               20
<210>  17
<211>  30
<212>  DNA
<213>  Artificial Sequence
<220>
<223>  Description of Artificial Sequence: primer
<400>  17
aacgcccatc agatcattag gaggctgtag                                    30
<210>  18
<211>  20
<212>  DNA
<213>  Artificial Sequence
<220>
<223>  Description of Artificial Sequence: primer
<400>  18
gatctgatgg gcgttcacgg                                               20
<210>  19
<211>  3224
<212>  DNA
<213>  Hepatitis B virus
<400>  19
tttttcacct ctgcctaatc atctcatgtt catgtcctac tgttcaagcc tccaagctgt      60
gccttgggtg gctttggggc atggacattg acccgtataa agaatttgga gcttctgtgg     120
agttactctc ttttttgcct tctgacttct ttccttctat tcgagatctc ctcgacaccg     180
cctctgctct gtatcgggag gccttagagt ctccggaaca ttgttcacct caccatacgg     240
cactcaggca agctattctg tgttggggtg agttaatgaa tctagccacc tgggtgggaa     300
gtaatttgga agatccagca tccagggaat tagtagtcag ctatgtcaac gttaatatgg     360
gcttaaaaat cagacaacta ttgtggtttc acatttcctg tcttactttt gggagagaaa     420
ctgttcttga atatttggtg tcttttggag tgtggattcg cactcctcct gcatatagac     480
cacaaaatgc ccctatctta tcaacacttc cggaaactac tgttgttaga cgaagaggca     540
ggtcccctag aagaagaact ccctcgcctc gcagacgaag gtctcaatcg ccgcgtcgca     600
gaagatctca atctcgggaa tctcaatgtt agtattcctt ggacacataa ggtgggaaac     660
tttacggggc tttattcttc tacggtacct tgctttaatc ctaaatggca aactccttct     720
tttcctgaca ttcatttgca ggaggacatt gttgatagat gtaagcaatt tgtggggccc     780
cttacagtaa atgaaaacag gagactaaaa ttaattatgc ctgctaggtt ttatcccaat     840
gttactaaat atttgccctt agataaaggg atcaaaccgt attatccaga gtatgtagtt     900
aatcattact tccagacgcg acattattta cacactcttt ggaaggcggg gatcttatat     960
aaaagagagt ccacacgtag cgcctcattt tgcgggtcac catattcttg ggaacaagat    1020
ctacagcatg ggaggttggt cttccaaacc tcgaaaaggc atggggacaa atctttctgt    1080
ccccaatccc ctgggattct tccccgatca tcagttggac cctgcattca aagccaactc    1140
agaaaatcca gattgggacc tcaacccgca caaggacaac tggccggacg ccaacaaggt    1200
gggagtggga gcattcgggc cagggttcac ccctccccat gggggactgt tggggtggag    1260
ccctcaggct cagggcctac tcacaactgt gccagcagct cctcctcctg cctccaccaa    1320
tcggcagtca ggaaggcagc ctactccctt atctccacct ctaagggaca ctcatcctca    1380
ggccatgcag tggaactcca ccactttcca ccaaactctt caagatccca gagtcagggc    1440
cctgtacttc cctgctggtg gctccagttc aggaacagtg agccctgctc agaatactgt    1500
ctctgccata tcgtcaatct tatcgaagac tggggaccct gtaccgaaca tggagaacat    1560
cgcatcagga ctcctaggac ccctgctcgt gttacaggcg gggtttttct tgttgacaaa    1620
aatcctcaca ataccacaga gtctagactc gtggtggact tctctcaatt ttctaggggg    1680
aacacccgtg tgtcttggcc aaaattcgca gtcccaaatc tccagtcact caccaacctg    1740
ttgtcctcca atttgtcctg gttatcgctg gatgtgtctg cggcgtttta tcatcttcct    1800
ctgcatcctg ctgctatgcc tcatcttctt gttggttctt ctggactatc aaggtatgtt    1860

gcccgtttgt cctctacttc caggatcatc aacgaccagc accggaccat gcaaaacctg 1920
cacaactcct gctcaaggaa cctctatgtt tccctcatgt tgctgtacaa aacctacgga 1980
cggaaactgc acctgtattc ccatcccatc atcttgggct ttcgcaaaat acctatggga 2040
gtgggcctca gtccgtttct cttggctcag tttactagtg ccatttgttc agtggttcgt 2100
agggctttcc cccactgtct ggctttcagt tatatggatg atgtggtttt gggggccaag 2160
tctgtacaac atcttgaatc cctttatgcc gctgttacca atttcttttt gtctttgggt 2220
atacatttaa accctcacaa aacaaaaaga tggggatatt cccttaactt catgggatat 2280
gtaattggga gttggggcac attgccacaa gaacatattg tacaaaaaat caaaatgtgt 2340
tttaggaaac ttcctgtaaa caggcctatt gattggaaag tatgtcaacg aattgtgggt 2400
cttttggggt ttgccgcccc tttcacgcaa tgtggatatc ctgctttaat gcctttatat 2460
gcatgtatac aagcaaaaca ggcttttact ttctcgccaa cttacaaggc ctttctaagt 2520
aaacagtatc tgaaccttta ccccgttgct cggcaacggc ctggtctgtg ccaagtgttt 2580
gctgatgcaa cccccactgg ttggggcttg gccataggcc atcagcgcat gcgtggaacc 2640
tttgtgtctc ctctgccgat ccatactgcg gaactcctag ccgcttgttt tgctcgcagc 2700
aggtctgggg caaaactcat cgggactgac aattctgtcg tgctctcccg caagtataca 2760
tcatttccat ggctgctagg ttgtgctgcc aactggatcc tgcgcgggac gtcctttgtt 2820
tacgtcccgt cggcgctgaa tcccgcggac gacccctccc ggggccgctt ggggctctac 2880
cgcccgcttc tccgcctgtt gtaccgaccg accacggggc gcacctctct ttacgcggac 2940
tccccgtctg tgccttctca tctgccggac cgtgtgcact tcgcttcacc tctgcacgtc 3000
gcatggagac caccgtgaac gcccacggga acctgcccaa ggtcttgcat aagaggactc 3060
ttggacccaa ggtcttgcat aagaggactc ttggactttc agcaatgtca acgaccgacc 3120
ttgaggcata cttcaaagac tgtgtgttta acgagtggga ggagttgggg gttgtactag 3180
gaggctgtag gcataaattg gtgtgttcac cagcaccatg caac 3224
<210> 20
<211> 30
<212> DNA
<213> Artificial Sequence
<220>
<223> Description of Artificial Sequence: primer
<400> 20
tagttctaga accctcacaa aacaaaaaga 30
<210> 21
<211> 30
<212> DNA
<213> Artificial Sequence
<220>
<223> Description of Artificial Sequence: primer
<400> 21
tagtgtcgac tttatacggg tcaatgtcca 30
<210> 22
<211> 30
<212> DNA
<213> Artificial Sequence
<220>
<223> Description of Artificial Sequence: primer
<400> 22
cctgctttaa tgcctccgat ccatactgcg 30
<210> 23
<211> 20
<212> DNA
<213> Artificial Sequence
<220>
<223> Description of Artificial Sequence: primer
<400> 23
aggcattaaa gcaggatatc 20
<210> 24
<211> 30
<212> DNA
<213> Artificial Sequence
<220>
<223> Description of Artificial Sequence: primer
<400> 24
aagaggactc ttggaaggct gtaggcataa 30

<210>  25
<211>  20
<212>  DNA
<212>  DNA
<213>  Artificial Sequence
<220>
<223>  Description of Artificial Sequence: primer
<400>  25
tccaagagtc ctcttatgca                                                    20
<210>  26
<211>  30
<212>  DNA
<212>  DNA
<213>  Artificial Sequence
<220>
<223>  Description of Artificial Sequence: primer
<400>  26
tagttctaga accctaacaa aacaaagaga                                         30
<210>  27
<211>  30
<212>  DNA
<212>  DNA
<213>  Artificial Sequence
<220>
<223>  Description of Artificial Sequence: primer
<400>  27
tagtgtcgac tttataaggg tcgatgtcca                                         30
<210>  28
<211>  30
<212>  DNA
<212>  DNA
<213>  Artificial Sequence
<220>
<223>  Description of Artificial Sequence: primer
<400>  28
cctgcgttaa tgcccccgat ccatactgcg                                         30
<210>  29
<211>  20
<212>  DNA
<212>  DNA
<213>  Artificial Sequence
<220>
<223>  Description of Artificial Sequence: primer
<400>  29
gggcattaac gcaggataac                                                    20
<210>  30
<211>  30
<212>  DNA
<212>  DNA
<213>  Artificial Sequence
<220>
<223>  Description of Artificial Sequence: primer
<400>  30
acgcccaccg aatgtactag gaggctgtag                                         30
<210>  31
<211>  20
<212>  DNA
<212>  DNA
<213>  Artificial Sequence
<220>
<223>  Description of Artificial Sequence: primer
<400>  31
acattcggtg ggcgttcacg                                                    20

## Description

### Technical Field

The present invention relates to a pharmaceutical composition having an anti-hepatitis B virus activity and a method of screening a substance that inhibits the production of a hepatitis B virus protein.

### Background Art

Hepatitis B virus (HBV) is a virus belonging to the genus Hepadnavirus and is a spherical DNA virus having a diameter of about 42 nm. The virus itself is a DNA virus consisting of a core having a diameter of 27 nm, which contains a coat (an envelope), an incomplete double-stranded DNA, a DNA polymerase, a reverse transcriptase, and the like, and is called a Dane particle. The Hepatitis B virus is formed in a double structure in which the outside consists of a hepatitis B surface (HBs) antigen (HBsAg) and the inside consists of a hepatitis B core antigen (HBcAg) and a genetic DNA (Non-Patent Document 1).

In addition to the Dane particle, the HBs antigen is present as a hollow particle, a small spherical particle, or a rod-shaped particle.

The HBV genome DNA includes four kinds of open reading frames (ORFs) of a pre-S/S gene region, a P gene region, an X gene region, and a pre-C/C gene region, three promoters, and two enhancer (Enh) regions. The core promoter of the X gene region and the precore region of the C gene region are involved in the production of a hepatitis B envelope antigen (an HBe antigen, HBeAg) constituent protein, and the point mutation of this region makes the production of the HBe antigen constituent protein impossible, which results in the HBe antigen negativity.

The two enhancer regions are called an enhancer I (Enh I) and an enhancer II (Enh II), and both of them can increase the promoter activity of all of the above three promoters (Patent Document 1).

In a case where HBV invades hepatocytes, the incomplete circular double-stranded DNA is incorporated into the nucleus of the hepatocytes and converted to a completely closed double-stranded DNA (a covalently closed circular DNA, cccDNA). In the state of chronically being infected by HBV, the cccDNA is present as a mini-chromosome at a quantity of 5 to 50 mini-chromosomes per hepatocyte. Four kinds of RNAs are transcribed from the cccDNA in the hepatocyte nucleus, from which the HBs antigen, the HBc antigen, the HBe antigen, and the X protein, as structural proteins, and polymerases including a reverse transcriptase are translated. One of the RNAs is incorporated into a core particle as the pregenomic RNA, a minus-strand DNA is synthesized by the action of the reverse transcriptase, and then a plus-strand DNA is synthesized to become an incomplete circular double-stranded DNA. Further, the incomplete circular double-stranded DNA is enveloped in an envelope formed of the HBs antigen to become a virus particle (a Dane particle), which is released into the blood. The hollow particle (the particle not having a nucleus with DNA) containing the HBs antigen, the HBc antigen, and the p22cr antigen, which are translated from mRNAs, the HBe antigen that passes through the hepatocyte membrane are released and secreted in a large amount into the blood separately from the Dane particle blood release route. This action is conceived to be an action of escaping the attack of the host immune system against the HBV infection. The HBe antigen, the HBc antigen, or the p22cr antigen can be measured as hepatitis, B core-related antigen (an HBcr antigen, HBcrAg) and is used as a marker of virus replication (Non-Patent Document 1).

Hepatitis B is a kind of viral hepatitis caused by infection with HBV, and the number of infected people is said to be about 240 million worldwide. As described above, in a case of being infected with HBV, Dane particles are released into the blood, and a large amount of the viral protein is secreted into the blood separately from the release route of the Dane particles. It has been pointed out that among viral proteins, the HBs antigen, in particular, may interfere with the elimination of infected cells by the host immune system (Non-Patent Documents 2 and 3). Accordingly, in the medical treatment of hepatitis B, it is conceived important to reduce Dane particles, that is, to suppress the proliferation of the virus and to reduce viral proteins such as the HBs antigen.

Nucleic acid analogs such as entecavir and tenofovir are used as the first-choice drugs for hepatitis B. A nucleic acid analog pharmaceutical preparation can reduce the number of Dane particles in the blood, that is, suppress the proliferation of the virus, by inhibiting the activity of the HBV polymerase protein and inhibiting the generation of the incomplete circular double-stranded DNA. However, since the nucleic acid analog pharmaceutical preparation cannot reduce the HBs antigen, it is difficult to achieve the elimination of infected cells by the host immune system. For this reason, it has been desired to develop a pharmaceutical drug capable of achieving the reduction of the HBs antigen.

In recent years, research and development of double-stranded RNA (dsRNA) that binds to an HBV RNA and degrades it based on the principle of RNA interference (RNAi) has been promoted. It has been reported that these double-stranded RNAs reduce the HBs antigen in HBV-infected cells and the like (Non-Patent Document 4). Due to its nature, a double-stranded RNA needs to have a high sequence identity with an HBV RNA in order to bind to the HBV RNA and to exhibit its effect. However, since there are eight types of genotypes in HBV and the RNA sequence changes by the gene mutation or the like, there is concern that the effect of a double-stranded RNA that binds to an HBV RNA cannot be expected. In fact, as a result of analyzing HBV collected from hepatitis B patients, it was reported that there are multiple clones having different gene sequences in the same patient and that there are clones in which the effect of a double-stranded RNA on the HBV RNA is diminished (Non-Patent Document 5). Accordingly, it is necessary to develop a pharmaceutical drug capable of achieving the reduction of the HBs antigen by a novel mechanism of action.

HBV does not have the group of molecules that are needed to transcribe RNAs from genes thereof, and the group of molecules that are needed to produce viral proteins from RNAs. Accordingly, the group of molecules of the infected human hepatocytes is required to be used for the HBV proliferation and the viral protein production. Therefore, a new approach in which a human protein that is used by HBV is targeted to inhibit the proliferation of HBV or the production of a viral protein has begun to attract attention.

For example, an attempt has been made to control transcription from an HBV genome DNA to an HBV RNA and regulate the HBV replication using a nucleic acid molecule that binds to the enhancer I in the HBV genome DNA (Patent Document 2). In addition, attempts have been made to control the transcription to an HBV RNA and suppress the HBV proliferation by controlling the expression of a transcription factor that acts on an enhancer region in the HBV genome DNA (Patent Documents 3 to 5).

However, it is not fully revealed which factors in the host human are required for the HBV proliferation and the viral protein production and what anti-HBV effects are exhibited by controlling such factors.

### Prior Art Documents

### Patent Documents

Patent Document 1: WO2005-059138A
Patent Document 2: US2003/0148985A
Patent Document 3: JP2018-526332A
Patent Document 4: ,JP2007-520461A
Patent Document 5: JP2005-532052A

### Non-Patent Documents

Non-Patent Document 1: Tanaka et al., Modern Media, Vol. 54, No. 12, pp. 347 to 352, 2008
Non-Patent Document 2: Brouw et al., Immunology, Vol. 126, pp. 280 to 289, 2008
Non-Patent Document 3: Vanlandschoot et al., Journal of General Virology, Vol. 83, pp. 1281 to 1289, 2002
Non-Patent Document 4: Wooddell et al., Molecular Therapy, Vol. 21, No. 5, pp. 973 to 985, 2013
Non-Patent Document 5: Wu et al., Gastroenterology, Vol. 128, pp. 708 to 716, 2005

### SUMMARY OF THE INVENTION

An object of the present invention is to provide a novel pharmaceutical composition that targets a human protein as a host factor and has an anti-hepatitis B virus (HBV) activity. In addition, another object of the present invention is to provide a pharmaceutical composition that targets a human protein and suppresses the production of an HBV protein. Further, an object of the present invention is to provide a method of screening a substance that targets a host factor and suppresses the production of an HBV protein.

As a result of the diligent research on the above-described problems, the inventors of the present invention have found that a pharmaceutical composition inhibiting a human protein that binds to at least one region of hepatitis B virus RNA (HBV RNA) sequences corresponding to an enhancer I (Enh I) region sequence or an enhancer II (Enh II) region sequence, on the hepatitis B virus genome DNA (the HBV genome DNA), has an ability to inhibit the production of an HBV protein, and the present invention has been completed.

That is, the present invention provides the followings.
<1> A pharmaceutical composition that inhibits a production of a hepatitis B virus protein (an HBV protein), in which the pharmaceutical composition inhibits an expression or a function of an RNA-binding protein that binds to at least one sequence selected from an HBV RNA sequence corresponding to an enhancer (Enh) I region sequence on an HBV genome DNA or an HBV RNA sequence corresponding to an enhancer (Enh) II region sequence on the HBV genome DNA.
<2> The pharmaceutical composition according to <1>, in which the HBV RNA sequence corresponding to the enhancer (Enh) I region sequence on the HBV genome DNA is a sequence having 80% or more of a sequence identity with SEQ ID NO: 1, and the HBV RNA sequence corresponding to the enhancer (Enh) II region sequence on the HBV genome DNA is a sequence having 80% or more of a sequence identity with SEQ ID NO: 2.
<3> The pharmaceutical composition according to <1>, in which the HBV RNA sequence corresponding to the enhancer (Enh) I region sequence on the HBV genome DNA is a sequence having 90% or more of a sequence identity with SEQ ID NO: 1, and the HBV RNA sequence corresponding to the enhancer (Enh) II region sequence on the HBV genome DNA is a sequence having 90% or more of a sequence identity with SEQ ID NO: 2.
<4> The pharmaceutical composition according to any one of <1> to <3>, in which the HBV protein is an HBs antigen protein.
<5> The pharmaceutical composition according to any one of <1> to <4>, in which the pharmaceutical composition reduces an expression level of the HBV genome DNA.
<6> The pharmaceutical composition according to any one of <1> to <5>, in which the pharmaceutical composition reduces an expression level of a completely closed double-stranded DNA (a cccDNA).
<7> The pharmaceutical composition according to any one of <1> to <6>, in which the RNA-binding protein is at least one protein selected from RBFOX1, ELAVL2, MBNL1, RBMX, SRSF9, SRSF10, SNRPA, ELAVL1, PUM2, YTHDC1, SRSF1, KHDRBS3, or EIF4B.
<8> A screening method which is a method of screening a substance that inhibits a production of an HBV protein, the method comprising identifying a substance that inhibits an expression or a function of an RNA-binding protein that binds to at least one sequence selected from an HBV RNA sequence corresponding to an enhancer (Enh) I region sequence on an HBV genome DNA or an HBV RNA sequence corresponding to an enhancer (Enh) II region sequence on the HBV genome DNA.
<9> The screening method according to <8>, in which the HBV RNA sequence corresponding to the enhancer (Enh) I region sequence on the HBV genome DNA is a sequence having 80% or more of a sequence identity with SEQ ID NO: 1, and the HBV RNA sequence corresponding to the enhancer (Enh) II region sequence on the HBV genome DNA is a sequence having 80% or more of a sequence identity with SEQ ID NO: 2.
<10> The screening method according to <8> or <9>, in which the HBV protein is an HBs antigen protein.
<11> A method of screening a substance that inhibits a production of a hepatitis B virus (HBV) protein, the method comprising;
   (a) a step of identifying an RNA-binding protein that binds to at least one sequence selected from an HBV RNA sequence corresponding to an enhancer (Enh) I region sequence on an HBV genome DNA or an HBV RNA sequence corresponding to an enhancer (Enh) II region sequence on the HBV genome DNA,
   (b) a step of bringing cells infected with HBV or cells expressing HBV into contact with a test substance that inhibits a function or an activity of an identified RNA-binding protein or with a test substance that reduces an expression level of the protein,
   (c) a step of measuring an ability of producing an HBV protein in the cells infected with HBV or the cells expressing HBV, and
   (d) a step of selecting a substance having an activity of inhibiting a production of the HBV protein of the step (c).
<12> The method according to <11>, in which the (a) includes a step of using an RNA-binding protein sequence database.
<13> The method according to <11>, in which the test substance of the (b) is an antibody, a peptide, a protein, a non-peptide compound, a synthetic compound, an antisense nucleic acid, a double-stranded RNA, an adeno-associated virus vector, or a CRISPR-Cas vector system.
<14> The method according to <11>, in which the (c) includes a step of measuring an HBV protein in a culture supernatant of the cells infected with HBV or the cells expressing HBV.
<15> The method according to <11>, in which the substance of the step (d) is an antibody, a peptide, a protein, a non-peptide compound, a synthetic compound, an antisense nucleic acid, a double-stranded RNA, an adeno-associated virus vector, or a CRISPR-Cas vector system.

Further, the present invention provides the followings.
<a> A treatment method comprising administering the pharmaceutical composition according to any one of <1> to <7> to a subject who is infected with HBV or has a disease associated with HBV infection.
<b> The treatment method according to <a>, in which the disease associated with HBV infection is hepatitis B, liver cirrhosis caused by hepatitis B, or liver cancer caused by hepatitis B or liver cirrhosis.
<c> A kit for treating a disease associated with HBV infection, the kit comprising:
   the pharmaceutical composition according to any one of <1> to <7>; and
   an instruction in which a treatment method for a disease associated with HBV infection is described.
<d> The kit for treating a disease associated with HBV infection according to <c>, in which the treatment method for a disease associated with HBV infection includes administering the pharmaceutical composition to a subject who is infected with HBV or has a disease associated with HBV infection.
<e> A substance for use in a treatment of inhibiting a production of a hepatitis B virus protein (an HBV protein), in which the substance inhibits an expression or a function of an RNA-binding protein that binds to at least one sequence selected from an HBV RNA sequence corresponding to an enhancer (Enh) I region sequence on an HBV genome DNA or an HBV RNA sequence corresponding to an enhancer (Enh) II region sequence on the HBV genome DNA.
<f> Use of a substance for producing a pharmaceutical composition that inhibits a production of a hepatitis B virus protein (an HBV protein), in which the substance inhibits an expression or a function of an RNA-binding protein that binds to at least one sequence selected from an HBV RNA sequence corresponding to an enhancer (Enh) I region sequence on an HBV genome DNA or an HBV RNA sequence corresponding to an enhancer (Enh) II region sequence on the HBV genome DNA.

The pharmaceutical composition according to the aspect of the present invention has an excellent anti-HBV activity and is useful as an anti-HBV agent. In addition, the screening method according to the aspect of the present invention is useful for screening a substance that inhibits the production of an HBV protein.

### BRIEF DESCRIPTION OF THE DRAWINGS

Fig. 1 shows the relative luminescence level (%) of the firefly luciferase in a reporter assay using an Enh I/Enh II region-deficient construct, where the Enh I/Enh II region is derived from the genotype C.
Fig. 2 shows the relative expression level (%) of the firefly luciferase RNA in a reporter assay using an Enh I/Enh II region-deficient construct, where the Enh I/Enh II region is derived from the genotype C.
Fig. 3 shows the relative value (%) of the HBs antigen (the HBsAg) of each of dsRNA-added wells in each concentration to the negative control dsRNA-added well.
Fig. 4 shows the relative value (%) of cell viability in each of the dsRNA-added wells in each concentration to the negative control dsRNA-added well.
Fig. 5 shows the relative expression level (%) of the firefly luciferase RNA and the relative luminescence level of the firefly luciferase in reporter assays using Enh I/Enh II region-deficient constructs, where the Enh I/Enh II regions are derived from various genotypes.

### DESCRIPTION OF THE PREFERRED EMBODIMENTS

Hereinafter, the present invention will be described in detail.

In the present specification, unless otherwise specified, each term has the following meaning.

The prevention means the inhibition of the onset of a disease, the reduction of the risk of the onset of a disease, or the delay of the onset of a disease.

The medical treatment means the amelioration or suppression of progression of a disease or condition of interest.

The treatment means the prevention or medical treatment of various diseases.

The effective amount means a therapeutically effective amount, a preventive effective amount, or the like.

Therapeutically effective amount is an amount sufficient to stabilize HBV infection, reduce HBV infection, or eradicate HBV infection in an infected subject. In addition, the preventive effective amount is an amount sufficient for preventing HBV infection in a subject under the risk of infection.

The subject means a mammal including a human.

### <Hepatitis B virus (HBV)>

Hepatitis B virus (HBV) means a virus that has the ability to develop hepatitis B. HBV is currently classified into eight genotypes, from A type to H type, based on the difference in the base sequence, which is derived from the gene mutation. The HBV to be prevented or medically treated with the pharmaceutical composition according to the embodiment of the present invention includes all genotypes thereof.

In the present invention, as a disease associated with HBV infection, hepatitis B is mentioned, and examples thereof include acute hepatitis, chronic hepatitis, and fulminant hepatitis. In addition, liver cirrhosis, liver fibrosis, and liver cancer such as hepatocellular carcinoma are also included in a case where the disease is caused by HBV infection to a living body including a human.

### <Hepatitis B virus protein (HBV protein)>

The hepatitis B virus protein (the HBV protein) is a protein constituting HBV, and examples thereof include an HBs antigen, an HBc antigen, and an HBe antigen.

In the present invention, the HBV protein is not particularly limited; however, it is preferably an HBs antigen.

### <HBV RNA sequence corresponding to Enh I region sequence and Enh II region sequence on HBV genome DNA>

The HBV genome DNA has two enhancer regions (Enh I and Enh II) thereon, and Enh I and Enh II are known to promote transcription from the HBV genome DNA to the HBV RNA.

In the present invention, in the HBV RNA sequence, the HBV RNA sequence corresponding to an Enh I region sequence and an Enh II region sequence, on the HBV genome DNA, means a base sequence of a region corresponding to Enh I on the HBV genome DNA, which is present in the 3' untranslated region (hereinafter also referred to as 3'UTR) of the RNA encoding an HBV protein, or a base sequence of a region corresponding to Enh II on the HBV genome DNA, which is present in the 3'UTR of the RNA encoding an HBV protein.

In the present invention, in the HBV RNA sequence, the HBV RNA sequence corresponding to an Enh I region sequence and an Enh II region sequence, on the HBV genome DNA, is preferably a base sequence of a region corresponding to EnhI on the HBV genome DNA, which is present in the 3'UTR of the RNA encoding an HBs antigen, or a base sequence of a region corresponding to EnhI on the HBV genome DNA, which is present in the 3'UTR of the RNA encoding the HBs antigen.

In the present invention, as the HBV RNA sequence corresponding to the Enh I region sequence on the HBV genome DNA, a sequence having 80% or more of a sequence identity with SEQ ID NO: 1 is mentioned, and as the HBV RNA sequence corresponding to the Enh II region sequence on the HBV genome DNA, a sequence having 80% or more of a sequence identity with SEQ ID NO: 2 is mentioned.

The above HBV RNA is an RNA encoding an HBV protein and is preferably an RNA encoding the HBs antigen.

Examples of the HBV RNA sequence include a sequence of SEQ ID NO: 1 or SEQ ID NO: 2. The above sequence preferably has 80% or more of a sequence identity, more preferably 85% of a sequence identity, still more preferably 90% of a sequence identity, even more preferably 95% of a sequence identity, even still more preferably 98% of a sequence identity, and particularly preferably 100% of a sequence identity, with the sequence of SEQ ID NO: 1 or SEQ ID NO: 2.
SEQ ID NO: 1: 
uuauaugcauguauacaaucuaagcaggcuuucacuuucucgccaacuuacaaggccuuucuguguaaacaau
aucugaaccuuuaccccguugcccggcaacggucaggucucugccaaguguuugcugacgcaacccccacuggauggggc
 uuggcuaucggccaucgccgcaugcguggaaccuuuguggcuccgcug
SEQ ID NO: 2:

uugcccaaggucuuauauaagaggacucuuggacucucagcgaugucaacgaccgaccuugaggcauacuuca
aagacuguuuguuuaaggacugggaggaguugggggaggagauuagguuaaagauuuuugu

### <RNA-binding protein>

An RNA-binding protein binds to a specific target RNA in a sequence-specific manner, and it is known to regulate various cell functions after transcription and in post-transcriptional protein expression, such as RNA splicing, polyadenylation, capping, modification, transport, localization, translation, and metabolic turnover (Ray et al., Nature, Vol. 499, No. 11, 2013).

In the present invention, it is presumed that the RNA-binding protein controls the expression (the production) of an HBV protein by binding to a specific sequence on the HBV RNA.

The HBV RNA is preferably an RNA encoding the HBs antigen.

Examples of the specific sequence on the HBV RNA include the sequence of SEQ ID NO: 1 or SEQ ID NO: 2. The above sequence preferably has 80% or more of a sequence identity, more preferably 85% of a sequence identity, still more preferably 90% of a sequence identity, even more preferably 95% of a sequence identity, even still more preferably 98% of a sequence identity, and particularly preferably 100% of a sequence identity, with the sequence of SEQ ID NO: 1 or SEQ ID NO: 2.

In the present invention, the RNA-binding protein preferably recognizes and binds to 4 to 9 bases on the above sequence, and examples thereof include RNA binding protein, fox-1 homolog (RBFOX1), ELAV like RNA binding protein 2 (ELAVL2), muscleblind-like Protein 1 (MBNL1), RNA-binding motif protein, X chromosome (RBMX), serine and arginine rich splicing factor 9 (SRSF9), serine and arginine rich splicing factor 10 (SRSF10), small nuclear ribonucleoprotein polypeptide A (SNRPA), ELAV like RNA binding protein 1 (ELAVL1), pumilio homolog 2 (PUM2), YTH domain containing 1 (YTHDC1), serine and arginine rich splicing factor 1 (SRSF1), KH RNA binding domain containing, signal transduction associated3 (KHDRBS3), and eukaryotic translation initiation factor 4B (EIF4B).

The RNA-binding protein of the present invention is preferably at least one protein selected from RBFOX1, ELAVL2, MBNL1, RBMX, SRSF9, SRSF10, SNRPA, ELAVL1, PUM2, YTHDC1, SRSF1, KHDRBS3, or EIF4B.

### <Pharmaceutical composition>

The pharmaceutical composition according to the embodiment of the present invention is a pharmaceutical composition containing a "substance", specifically a "substance that inhibits the production of an HBV protein", and a pharmaceutically acceptable carrier.

The pharmaceutical composition according to the embodiment of the present invention contains, as a substance that inhibits the production of an HBV protein, a substance that inhibits the expression or the function of an RNA-binding protein that binds to at least one sequence selected from an HBV RNA sequence corresponding to an enhancer (Enh) I region sequence on an HBV genome DNA or an HBV RNA sequence corresponding to an enhancer (Enh) II region sequence on the HBV genome DNA.

The pharmaceutical composition according to the embodiment of the present invention preferably has a substance that inhibits the production of an HBV protein and more preferably a substance that inhibits the production of the HBs antigen.

Examples of the "substance" include an antibody, a peptide, a protein, a non-peptide compound, a synthetic compound, an antisense nucleic acid, a double-stranded RNA, an adeno-associated virus vector, and a CRISPR-Cas vector system.

The "antisense nucleic acid" is a nucleic acid including a base sequence or a part there of, which is complementary or substantially complementary to a base sequence of a "sense" nucleic acid that encodes a protein, such as a coding strand of a double-stranded cDNA molecule, an mRNA sequence, or a coding strand of a gene, and binds to a target base sequence to form a specific and stable double strand, and thus has the function of inhibiting protein synthesis.

An "RNAi nucleic acid" includes, but is not limited to, a nucleic acid that interferes with or inhibits the expression of a target gene by a small interfering RNA (a siRNA), a short hairpin RNA (an shRNA), or RNA interference (RNAi).

A "double-stranded RNA" includes, but is not limited to, a nucleic acid that interferes with or inhibits the expression of a target gene by a short interfering RNA (a siRNA), a short hairpin RNA (an shRNA), or RNA interference (RNAi).

An "RNA interference agent" is a nucleic acid that interferes with or inhibits the expression of a target gene through RNA interference.

The RNA interference is a post-transcriptional target gene silencing technique that uses a double-stranded RNA (a dsRNA) to degrade an mRNA including the same sequence as the dsRNA (Sharp et al., Science, 287, 2431 to 2432, 2000); Zamore et al., Cell, Vol. 101, pp. 25 to 33, 2000; Tuschl et al., Genes & Development, Vol. 13, 3191 to 3197, 1999). The RNA interference occurs in a case where an endogenous ribonuclease cleaves a long dsRNA to generate a shorter RNA having a length of 21 or 22 nucleotides, called a short interfering RNA (siRNA). This siRNA mediates the degradation of a target mRNA. Synthesis kits for RNAi are commercially available, for example, from New England Biolabs and Ambion. In an aspect of the synthesis of RNAi, one or more of the above synthesis kits for use in antisense RNA can be used.

The substance contained in the pharmaceutical composition according to the embodiment of the present invention is preferably an antisense nucleic acid or a double-stranded RNA, and more preferably a double-stranded RNA.

Further, in the case of the double-stranded RNA, it is particularly preferably an RNA interference agent.

The "adeno-associated virus" is a single-stranded DNA virus, which belongs to the family Parvoviridae and consists of about 4,700 bases, has no envelope and has a capsid of a regular icosahedral structure having a diameter of 20 to 30 nm. Since the adeno-associated virus recognizes heparan sulfate proteoglycan which is a universal component of the cell membrane to infect a cell, the range of hosts thereof is wide.

Any gene or nucleic acid can be inserted into the genome of the adeno-associated virus. For this reason, it is possible to introduce a gene or nucleic acid of interest by infecting a target cell with a genetically modified adeno-associated virus, and thus it is possible to use the adeno-associated virus as a vector for gene transfer. Further, since the "adeno-associated virus vector" is non-pathogenic and enables the gene transfer into a terminally differentiated non-dividing cell, it is expected to be applied to gene therapy.

It is known that adeno-associated viruses have different infection directivity to tissues and cells depending on the difference in serotype (Ozawa et al., Drug Delivery System, Vol. 22, No. 6, pp. 643 to 650, 2007).

The substance contained in the pharmaceutical composition according to the embodiment of the present invention is preferably an adeno-associated virus type 5, an adeno-associated virus type 7, an adeno-associated virus type 8, or an adeno-associated virus type 9, and more preferably an adeno-associated virus type 8.

The "CRISPR-Cas system" is a system that uses, for genome editing, the clustered regulatory interspaced short palindromic repeats/CRISPR-associated protein (CRISPR/Cas) system discovered as acquired immunity of eubacteria and archaea (Jinek et al., Science, Vol. 337, pp. 816-821, 2012). Specifically, in the genomic region called the CRISPR region of the above bacteria, a plurality of cassettes in which a fragmented foreign DNA (20 bp) is incorporated are repeated, and a different foreign DNA is incorporated into each of the cassettes.

It is conceived that each of the cassettes is generated by fragmenting a DNA of a foreign introduced species (for example, a phage) and incorporating the fragmented DNA into the CRISPR region in a case where the DNA is incorporated into the cell.

Each of the cassettes encodes a CRISPR RNA (a crRNA). The crRNA has a protospacer sequence and a sequence complementary to the trans-crRNA (the tracrRNA) described later. The protospacer sequence has a length of 20 nucleotides and has a sequence complementary to the target DNA sequence. The protospacer sequence need not have the complementarity of 100% with the target sequence and is allowed to have mismatches in the region of 6 nucleotides from the 5' end. The crRNA forms a complex with the trans-crRNA (the tracrRNA), which is separately transcribed, through the complementary moiety. Further, the complex of the crRNA and the tracrRNA forms a complex with Cas9 endonuclease. The protospacer moiety in the crRNA forms a complementary hybrid with the foreign DNA, which leads the Cas9 endonuclease to the target sequence of the foreign DNA. The foreign DNA is cleaved by the Cas9 endonuclease at the site of the target sequence, and the CRISPR-Cas9 system protects the host from the foreign DNA.

As described above, the CRISPR-Cas9 system has been discovered as a system of eubacteria and archaea, which functions as acquired immunity to a new foreign DNA; however, the CRISPR-Cas9 system makes it possible to sequence-dependently cleave a mammalian genome by designing a protospacer consisting of a foreign DNA, whereby the mammalian genome can be edited.

The "CRISPR-Cas vector system" is a vector system containing a crRNA, a tracrRNA sequence, or a guide RNA (a gRNA) having both functions of the crRNA and the tracrRNA, and a Cas gene sequence. Currently, the predominantly used system is, but is not limited to, a second type CRISPR-Cas9 system, which is derived from Streptococcus pyogenes (S. pyogenes).

The pharmaceutically acceptable carrier includes an excipient, a diluent, a bulking agent, a disintegrating agent, a stabilizer, a preservative, a buffer, an emulsifier, a fragrance, a colorant, a sweetener, a thickening agent, a taste improving agent, a dissolution auxiliary agent, and other additives. In a case where one or more of such carriers are used, it is possible to prepare pharmaceutical compositions having forms such as a tablet, a capsule, a powdered drug, a syrup, a granule, a pill, a suspension, an emulsion, a powder preparation, a suppository, an eye drop, a nasal drop, an ear drop, a patch, an ointment, an injection agent, a liquid drug, a troche, and an elixir.

These pharmaceutical compositions can be administered orally or parenterally. Examples of other forms for parenteral administration include an external liquid drug that contains one or more active substances and is prescribed according to the rule and a suppository for enteric administration.

In addition, the dose and the frequency of administration of the pharmaceutical composition according to the embodiment of the present invention can be appropriately selected depending on the sex, weight, age, severity, symptom, and the like of the patient. In general, for adults, a daily dose of 0.01 to 1,000 mg/kg may be dividedly administered one to several times by oral or parenteral administration (for example, injection, drip infusion, or administration to the rectal region).

The pharmaceutical composition according to the embodiment of the present invention preferably further reduces the expression level of the HBV DNA.

The pharmaceutical composition according to the embodiment of the present invention preferably further reduces the expression level of the completely closed double-stranded DNA (the cccDNA).

The pharmaceutical composition according to the embodiment of the present invention is useful as a pharmaceutical drug for inhibiting the expression or the function of an RNA-binding protein that binds to at least one an HBV RNA sequence corresponding to an Enh I region sequence on an HBV genome DNA or an HBV RNA sequence corresponding to an Enh II region sequence on the HBV genome DNA, or as a pharmaceutical drug for suppressing or inhibiting the onset or progression of a disease in which the RNA-binding protein is involved and for medically treating or preventing the disease.

### <Screening method>

The screening method according to the embodiment of the present invention is useful as a method of screening a substance that inhibits the production of an HBV protein.

The screening method according to the embodiment of the present invention is a screening method of screening a substance that inhibits the production of an HBV protein and is useful as the screening method, where the method includes identifying a substance that inhibits an expression or a function of an RNA-binding protein that binds to at least one sequence selected from an HBV RNA sequence corresponding to an enhancer I region sequence on an HBV genome DNA or an HBV RNA sequence corresponding to an enhancer II region sequence on the HBV genome DNA.

In the screening method according to the embodiment of the present invention, it is preferable that the HBV RNA sequence corresponding to the enhancer I region sequence on the HBV genome DNA is a sequence having 80% or more of a sequence identity with SEQ ID NO: 1, and the HBV RNA sequence corresponding to the enhancer II region sequence on the HBV genome DNA is a sequence having 80% or more of a sequence identity with SEQ ID NO: 2.

In addition, in the screening method according to the embodiment of the present invention, it is preferable that a substance that inhibits the production of the HBs antigen as a hepatitis B virus protein is identified.

Examples of the screening method according to the embodiment of the present invention include a method including;
(a) a step of identifying an RNA-binding protein that binds to at least one sequence selected from an HBV RNA sequence corresponding to an Enh I region sequence on an HBV genome DNA or an HBV RNA sequence corresponding to an Enh II region sequence on the HBV genome DNA,
(b) a step of bringing cells infected with HBV or cells expressing HBV into contact with a test substance that inhibits a function or an activity of an identified RNA-binding protein or with a test substance that reduces an expression level of the protein,
(c) a step of measuring an ability of producing an HBV protein in the cells infected with HBV or the cells expressing HBV, and
(d) a step of selecting a substance having an activity of inhibiting a production of the HBV protein of the step (c).

### (a) A step of identifying an RNA-binding protein that binds to at least one sequence selected from an HBV RNA sequence corresponding to an Enh I region sequence on an HBV genome DNA or an HBV RNA sequence corresponding to an Enh II region sequence on the HBV genome DNA

For example, the utilization of the public RNA-binding protein sequence database such as RNA-Binding Protein DataBase, RBPDB, can be mentioned. The public RNA-binding protein sequence database to be utilized is not particularly limited. In a case where an HBV RNA sequence corresponding to the Enh I region sequence or the Enh II region sequence, on the HBV genome DNA, is input into RBPDB or a database similar thereto, an RNA-binding protein that binds to this region sequence can be extracted.

Further, for example, a method or a method similar thereto, in which the cell extract derived from HepG 2.2.15 cells and the in vitro transcribed RNA having a sequence corresponding to the Enh I region sequence or the Enh II region sequence, on the HBV genome DNA, are used to carry out a pull-down assay, and then the obtained binding proteins are identified with a mass spectrometer.

In the screening method according to the embodiment of the present invention, it is preferable that the (a) includes a step using the RNA-binding protein sequence database.

Further, as another aspect, it is preferable that (a) is a method including a step of carrying out a pull-down assay using the RNA having a sequence corresponding to the Enh I region sequence or the Enh II region sequence, on the HBV genome DNA.

### (b) A step of bringing cells infected with HBV or cells expressing HBV into contact with a test substance that inhibits a function or an activity of an identified RNA-binding protein or with a test substance that reduces an expression level of the protein

Examples of HBV-infected cells include a cultured human primary hepatocyte infected with HBV clone C_JPNAT (GenBank: AB246345.1), and a HepG2 cell forcibly expressing a sodium taurocholate coating polypeptide (NTCP), which is infected with HBV clone C_JPNAT (GenBank: AB246345.1). The combination of the HBV clone and the cell described above is not particularly limited.

Examples of the cells expressing HBV include a HepG2.2.15 cell, and a HepG2 cell, a Huh-7 cell, a HepaRG cell, and a cultured human primary hepatocyte, which forcibly express a plasmid formed by containing a 1.24-fold lengthened genome sequence of HBV clone C_JPNAT (GenBank: AB246345.1). The above HBV clone is not particularly limited. The length of the sequence derived from the HBV genome, which is inserted into the plasmid formed by containing the genome sequence of the HBV clone is not particularly limited as long as it is at least 1.24 folds the length of the genome sequence of the HBV clone.

Examples of the test substance include a substance selected from an antibody, a peptide, a protein, a non-peptide compound, a synthetic compound, an antisense nucleic acid, a double-stranded RNA, an adeno-associated virus vector, and a CRISPR-Cas vector system.

The test substance that is used in the screening method according to the embodiment of the present invention is preferably an antisense nucleic acid or a double-stranded RNA, and more preferably a double-stranded RNA.

Further, in the case of the double-stranded RNA, it is particularly preferably an RNA interference agent.

The contact between a cell infected with HBV or a cell expressing HBV and a test substance can be carried out, for example, by adding the test substance to a culture medium containing the above cell and culturing for a certain period of time. The concentration of the test substance to be added varies depending on the properties of substance (solubility, toxicity, and the like); however, it is appropriately selected in a range of, for example, about 0.1 nmol/L to about 100 nmol/L. Examples of the culturing time include about 24 hours to about 120 hours.

### (c) A step of measuring an ability of producing an HBV protein in the cells infected with HBV or the cells expressing HBV

Examples of the step of measuring the ability of producing HBV protein include a step of measuring an HBV protein in a culture supernatant of the cells infected with HBV or the cells expressing HBV.

The HBV protein can be measured using the collected culture supernatant, for example, by a chemiluminescence enzyme-linked immunosorbent assay (a CLEIA method) or an enzyme-linked immunosorbent assay (an ELISA method), or by a method similar thereto.

### (d) A step of selecting a substance having an activity of inhibiting a production of the HBV protein of the step (c)

For example, in a case where test substances are allowed to act in the (c), it is possible to select a substance that inhibits the production of the HBV protein by 30% or more, preferably 50% or more, more preferably 70% or more, still more preferably 90% or more, as compared with the negative control.

### <Use application of pharmaceutical composition>

The pharmaceutical composition according to the embodiment of the present invention is useful in the medical treatment or prevention of HBV infection.

The pharmaceutical composition according to the embodiment of the present invention can inhibit the production of an HBV protein, particularly the HBs antigen. In addition, the HBV gene expression (the HBV DNA production) can be inhibited. As a result, the pharmaceutical composition according to the embodiment of the present invention is useful for the medical treatment or prevention of HBV infection.

The present invention relates to the use of the pharmaceutical composition according to the embodiment of the present invention for inhibiting the production of the HBs antigen.

The present invention relates to the use of the pharmaceutical composition according to the embodiment of the present invention for inhibiting the DNA production of HBV.

The present invention relates to the use of the pharmaceutical composition according to the embodiment of the present invention for inhibiting the gene expression of HBV.

The present invention relates to the use of the pharmaceutical composition according to the embodiment of the present invention for the medical treatment or prevention of HBV infection.

The disease associated with HBV infection includes progressive liver fibrosis, inflammation, and necrosis, which progress to liver cirrhosis, end-stage liver disease, and hepatocellular carcinoma.

Next, the present invention will be described with reference to Test Examples; however, the present invention is not limited thereto.

### [Examples]

### [Test Example 1]

### (Reporter assay using Enh I/Enh II region-deficient construct)

PCR reaction was carried out in a reaction solution containing 1× PrimeSTAR Max DNA Polymerase (manufactured by Takara Bio Inc.) and a 0.2 µmol/L primer. PCR was carried out using an HBV genome DNA (GenBank: AB246345.1; hereinafter, also referred to as a "genotype C") as a template and using a primer combination of a primer consisting of the nucleotide sequence of SEQ ID NO: 3 and a primer consisting of the nucleotide sequence of SEQ ID NO: 4, under the condition of 35 cycles at 98°C for 10 seconds, 58°C for 5 seconds, and 72°C for 15 seconds, to obtain an amplified fragment of the region of 410 bp containing the PreS2/S promoter, in which restriction enzyme sites of Kpn I and Hind III were introduced. Similarly, PCR was carried out using a primer combination of a primer consisting of the nucleotide sequence of SEQ ID NO: 5 and a primer consisting of the nucleotide sequence of SEQ ID NO: 6, under the condition of 35 cycles at 98°C for 10 seconds, 58°C for 5 seconds, and 72°C for 15 seconds, to obtain an amplified fragment of the region of 1,086 bp containing the sequence of the 3' untranslated region (hereinafter, also referred to as "3'UTR") of the gene encoding the HBs antigen, in which restriction enzyme sites of Xba I and Sal I were introduced. The obtained fragments were respectively treated with a combination of Kpn I (manufactured by Takara Bio Inc.) and Hind III (manufactured by Takara Bio Inc.) and a combination of Xba I (manufactured by Takara Bio Inc.) and Sal I (manufactured by Takara Bio Inc.), and respectively inserted into Kpn I and Hind III sites and Xba I and Sal I sites of pGL3 (manufactured by Promega Corporation) to obtain a ligated plasmid. Hereinafter, this plasmid is also referred to as "PreS2/S-Luc-3'UTR". In Test Example 4, which will be described later, it is also referred to as "PreS2/S-Luc-3'UTR (genotype C)" in order to clarify the difference in genotype. PCR was carried out using the PreS2/S-Luc-3'UTR plasmid as a template and using a primer combination of a primer consisting of the nucleotide sequence of SEQ ID NO: 7 and a primer consisting of the nucleotide sequence of SEQ ID NO: 8 and a primer combination of a primer consisting of the nucleotide sequence of SEQ ID NO: 9 and a primer consisting of the nucleotide sequence of SEQ ID NO: 10, under the condition of 35 cycles at 98°C for 10 seconds, 58°C for 5 seconds, and 72°C for 30 seconds, to obtain amplified fragments for constructing an Enh I region-deficient plasmid. These fragments were ligated using an In-Fusion HD Cloning Kit (manufactured by Takara Bio Inc.) to obtain plasmids lacking the Enh I region in the PreS2/S-Luc-3'UTR plasmid. Hereinafter, this plasmid is also referred to as "PreS2/S-Luc-3'UTR dEnh I". PCR was carried out using the PreS2/S-Luc-3'UTR plasmid as a template and using a primer combination of a primer consisting of the nucleotide sequence of SEQ ID NO: 7 and a primer consisting of the nucleotide sequence of SEQ ID NO: 11 and a primer combination of a primer consisting of the nucleotide sequence of SEQ ID NO: 10 and a primer consisting of the nucleotide sequence of SEQ ID NO: 12, under the condition of 35 cycles at 98°C for 10 seconds, 58°C for 5 seconds, and 72°C for 30 seconds, to obtain amplified fragments for constructing an Enh II region-deficient plasmid. These fragments were ligated using an In-Fusion HD Cloning Kit (manufactured by Takara Bio Inc.) to obtain plasmids lacking the Enh II region in the PreS2/S-Luc-3'UTR plasmid. Hereinafter, this plasmid is also referred to as "PreS2/S-Luc-3'UTR dEnh II".

**[Table 1]**

| SEQ ID NO | Sequence (5' → 3') |
|---|---|
| 3 | GTCAGGTACCCAAACAATCCAGATTGGGAC |
| 4 | TGACAAGCTTGTTCGGTGCAGGGTCC |
| 5 | TGACTCTAGAACCCTAATAAAACCAAACGT |
| 6 | TGACGTCGACTTTATACGGGTCAATGTCCA |
| 7 | CGCGGGGCATGACTATCGTC |
| 8 | AGGCATTAAGGCAGGATAGC |
| 9 | CCTGCCTTAATGCCTCCGATCCATACTGCG |
| 10 | GACGATAGTCATGCCCCGCG |
| 11 | GACCTGGTGGGCGTTCACGG |
| 12 | AACGCCCACCAGGTCACTAGGAGGCTGTAG |

HepG 2.2.15 cells obtained by introducing the HBV genome into a hepatoblastoma cell line HepG2 (Proc Natl Acad Sci USA, Vol. 84, pp. 1005 to 1009, 1987), which are cells continuously producing viruses, were used, and the reporter assay was carried out using the following procedure.

The following medium was used as a cell culture medium.

DMEM/F-12, GlutaMAX (manufactured by Thermo Fisher Scientific, Inc.) + 5 µg/mL insulin (manufactured by FUJIFILM Wako Pure Chemical Corporation) + 1% penicillin/streptomycin (manufactured by Sigma-Aldrich Co., LLC) + 10 mmol/L HEPES (manufactured by Sigma-Aldrich Co., LLC) + 50 µmol/L hydrocortisone (manufactured by Sigma-Aldrich Co., LLC) +10 % FBS (manufactured by Equitech-Bio Inc.)

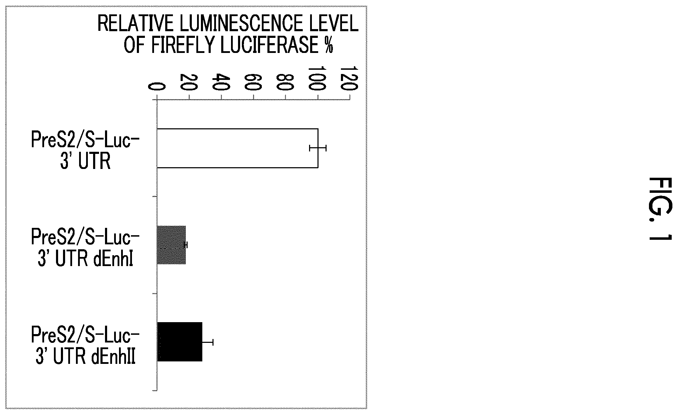

HepG 2.2.15 cells were suspended in the above medium, seeded on a 96-well microtiter plate so that the cell concentration was 1 × 10⁴ cells/100 µL per well, and incubated in a 5% CO₂ incubator at 37°C for 24 hours. 0.062 µg of any one of a PreS2/S-Luc-3'UTR plasmid, a PreS2/S-Luc-3'UTR dEnh I plasmid, or a PreS2/S-Luc-3'UTR dEnh II plasmid, 0.25 ng of a pRL-SV40 plasmid (manufactured by Promega Corporation), and 0.23 µL of TransIT-X2 Transfection Reagent (manufactured by Mirus Bio LLC) were diluted with 7.8 µL of serum-free medium (manufactured by Thermo Fisher Scientific, Inc.) and incubated at room temperature for 20 minutes. 8 µL of this plasmid-TransIT-X2 Transfection Reagent mixture was added to the wells seeded with HepG 2.2.15 cells described above and incubated for 24 hours. Next, the medium was exchanged at 100 µL/well with the above-described cell culture medium, and incubation was further carried out at 37°C for 48 hours in a 5% CO₂ incubator. After incubation, the luminescence by firefly luciferase and the luminescence by Renilla luciferase were detected in Dual-Luciferase Reporter Assay System (manufactured by Promega Corporation). Regarding each of the wells, the results of calculating the luminescence level of firefly luciferase corrected by the luminescence level of Renilla luciferase are shown in Fig. 1. In addition, the results of calculating the relative value (%) of the luminescence level of luciferase in each of the defective plasmid-added wells to the luminescence level of firefly luciferase in the PreS2/S-Luc-3'UTR plasmid-added well are shown in Fig. 1.

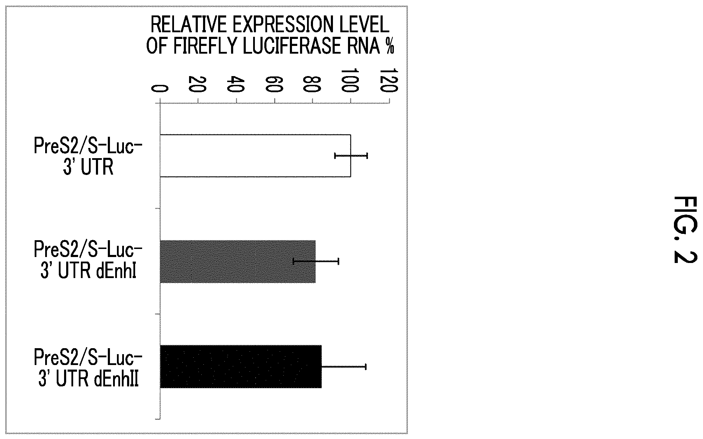

HepG 2.2.15 cells were suspended in the above medium, seeded on a 12-well microtiter plate so that the cell concentration was 2 × 10⁵ cells/1,000 µL per well, and incubated in a 5% CO₂ incubator at 37°C for 24 hours. 1 µg of any one of a PreS2/S-Luc-3'UTR plasmid, a PreS2/S-Luc-3'UTR dEnh I plasmid, or a PreS2/S-Luc-3'UTR dEnh II plasmid, 3 µL of TransIT-X2 Transfection Reagent (manufactured by Mirus Bio LLC) were diluted with 97 µL of serum-free medium (manufactured by Thermo Fisher Scientific, Inc.) and incubated at room temperature for 20 minutes. 100 µL of this plasmid-TransIT-X2 Transfection Reagent mixture was added to the wells seeded with HepG 2.2.15 cells described above and incubated for 24 hours. Next, the medium was exchanged at 1,000 µL/well with the above-described cell culture medium, and incubation was further carried out at 37°C for 24 hours in a 5% CO₂ incubator. Then, the culture supernatant was discarded, the cells were washed with phosphate buffered saline (1,000 µL/well, manufactured by FUJIFILM Wako Pure Chemical Corporation), and total RNA was extracted using an RNeasy Mini Kit (manufactured by Qiagen). 1 µg of the extracted total RNA was reverse transcribed using a PrimeScript RT Reagent Kit with gDNA Eraser (manufactured by Takara Bio Inc.) to prepare cDNA. The expression level of the firefly luciferase RNA was quantified based on the prepared cDNA, and the results of calculating the relative value (%) of the expression level of the firefly luciferase in each of the plasmid-added wells to the expression level of the firefly luciferase RNA in the PreS2/S-Luc-3'UTR plasmid-transfected well are shown in Fig. 2.

Due to the deletion of the Enh I region or the Enh II region, the relative luminescence level derived from the firefly luciferase protein was reduced by about 70% to 80% (Fig. 1). From this result, it was conceived that the above phenomenon may be caused by the decrease in transcriptional activity (the decrease in firefly luciferase RNA expression level) due to the deletion of the Enh I region or the Enh II region from the DNA sequence, or may be caused by the functional change of RNA due to the deletion of the region corresponding to Enh I or Enh II from the RNA sequence. For this reason, the relative expression level of the firefly luciferase RNA was analyzed, and as a result, the relative expression level of the firefly luciferase RNA was hardly changed due to the deletion of the Enh I region or the Enh II region (Fig. 2). From this, it has been revealed that the decrease in the relative luminescence level due to the deletion of the Enh I region or the Enh II region is caused by the functional change of RNA due to the deletion of the region corresponding to Enh I or the region corresponding to Enh II from the RNA sequence. That is, it has been revealed that the region corresponding to Enh I on the HBV genome DNA or the region corresponding to Enh II on the HBV genome DNA functions on the RNA and controls the expression level of the protein.

### [Test Example 2]

### (In silico identification of RNA-binding protein)

Since the region corresponding to Enh I and the region corresponding to Enh II function on the RNA, an attempt was made to identify an RNA-binding protein that binds to these regions. Putative RNA-binding proteins were identified by inputting an Enh I-equivalent region in 3'UTR (an RNA sequence corresponding to the Enh I region, consisting of base number 1,060 to 1,260 of GenBank: AB246345, SEQ ID NO: 1) of the RNA encoding the HBs antigen or an Enh II-equivalent region in 3'UTR (an RNA sequence corresponding to the Enh II region, consisting of base number 1,638 to 1,771 of GenBank: AB246345, SEQ ID NO: 2) of the RNA encoding the HBs antigen, to the public RNA-binding protein sequence database (the RNA-Binding Protein DataBase, RBPDB), and by executing a binding protein prediction program. RBPDB is a database where information on RNA-binding proteins and their binding sequences obtained from in-vitro and in-vivo experiments using a human, mice, flies, and the like, are accumulated, and in a case where an RNA base sequence is input, it is possible to extract RNA-binding proteins that are presumed to bind to an RNA having the input RNA sequence (Nucleic Acids Res, Vol. 39, pp. D301 to D308, 2011).

**[Table 2]**

| Proteins that bind to Enh I-equivalent region or Enh II-equivalent region on RNA encoding HBs antigen | | |
|---|---|---|
| Protein name | Enh I-equivalent region | Enh II-equivalent region |
| RBFOX1 | Yes | No |
| ELAVL2 | Yes | No |
| MBNL1 | Yes | No |
| RBMX | Yes | No |
| SRSF9 | No | Yes |
| SRSF10 | No | Yes |
| SNRPA | No | Yes |
| ELAVL1 | Yes | Yes |
| PUM2 | Yes | Yes |
| YTHDC1 | Yes | Yes |
| SRSF1 | Yes | Yes |
| KHDRBS3 | Yes | Yes |
| EIF4B | Yes | Yes |

It was expected that a plurality of RNA-binding proteins would bind to at least one region of the Enh I-equivalent region or the Enh II-equivalent region, on the RNA encoding the HBs antigen.

### [Test Example 3]

### (Anti-HBV assay)

HepG 2.2.15 cells obtained by introducing the HBV genome into a hepatoblastoma cell line HepG2 (Proc Natl Acad Sci USA, Vol. 84, pp. 1005 to 1009, 1987), which are cells continuously producing viruses, were used, and the anti-HBV effect obtained by the inhibition of each of molecules was evaluated by the following procedure.

The following medium was used as a cell culture medium.

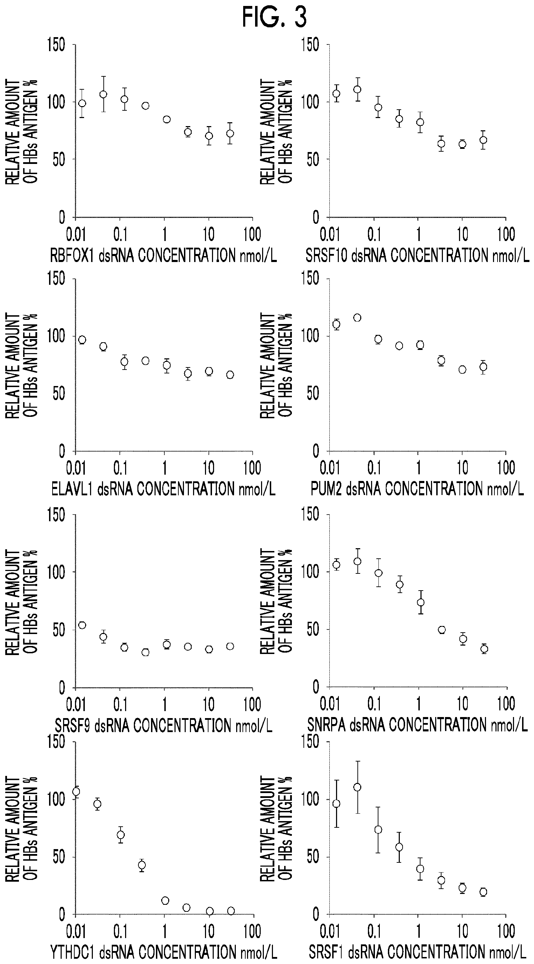

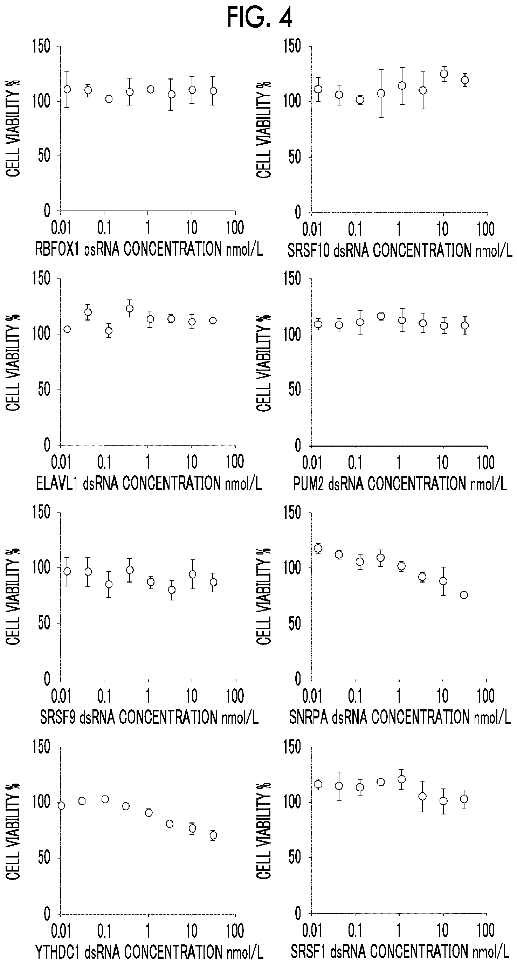

DMEM/F-12, GlutaMAX (manufactured by Thermo Fisher Scientific, Inc.) + 5 µg/mL insulin (manufactured by FUJIFILM Wako Pure Chemical Corporation) + 1% penicillin/streptomycin (manufactured by Sigma-Aldrich Co., LLC) + 10 mmol/L HEPES (manufactured by Sigma-Aldrich Co., LLC) + 50 µmol/L hydrocortisone (manufactured by Sigma-Aldrich Co., LLC) +10 % FBS (manufactured by Equitech-Bio Inc.)
(1) The dsRNA used for transfection was as follows (manufactured by Dharmacon Inc.). SMARTpool: ON-TARGETplus RBFOX1 siRNA (L-013377-01), SMARTpool: ON-TARGETplus ELAVL1 siRNA (L-003773-00), SMARTpool: ON-TARGETplus ELAVL2 siRNA (L-019801-00), SMARTpool: ON-TARGETplus MBNL1 siRNA (L-014136-00), SMARTpool: ON-TARGETplus RBMX siRNA (L-011691-01), SMARTpool: ON-TARGETplus PUM2 siRNA (L-014031-02), SMARTpool: ON-TARGETplus YTHDC1 siRNA (L-015332-02), SMARTpool: ON-TARGETplus SRSF1 siRNA (L-018672-01), SMARTpool: ON-TARGETplus SRSF9 siRNA (L-019529-01), SMARTpool: ON-TARGETplus SRSF10 siRNA (L-007278-00), SMARTpool: ON-TARGETplus KHDRBS3 siRNA (L-012748-01), SMARTpool: ON-TARGETplus EIF4B siRNA (L-020179-00), and SMARTpool: ON-TARGETplus SNRPA siRNA (L-019435-02). In addition, an ON-TARGETplus Non-targeting Control Pool (manufactured by Dharmacon Inc.) was used as a negative control dsRNA.
(2) HepG 2.2.15 cells were suspended in the above medium to prepare a HepG 2.2.15 cell suspension of 2 × 10⁵ cells/mL. The dsRNA was diluted with Nuclease-Free Water (not DEPC-Treated) (manufactured by Thermo Fisher Scientific, Inc.) to prepare dsRNA solutions of 3-fold dilution series from 2.743 nmol/L to 2,000 nmol/L. 1.5 µL of the prepared dsRNA solution and 0.3 µL of Lipofectamine RNAiMAX (manufactured by Thermo Fisher Scientific, Inc.) were diluted with 8.2 µL of serum-free medium (manufactured by Thermo Fisher Scientific, Inc.) and incubated at room temperature for 5 minutes. 10 µL of this dsRNA-Lipofectamine RNAiMAX mixture was diluted with 40 µL of a cell culture medium to adjust the volume to 50 µL. This diluted mixture was mixed with 50 µL of the above-described HepG 2.2.15 cell suspension, seeded on a 96-well microtiter plate, and incubated in a 5% CO₂ incubator at 37°C for 3 days (final concentration of dsRNA: 0.91 to 30 nmol/L, 3-fold dilution series). Next, the medium was exchanged at 100 µL/well with the above-described cell culture medium, and incubation was further carried out at 37°C for 3 days in a 5% CO₂ incubator. Then, the culture supernatant was collected, and the cell viability was measured using CellTiter96 AQueous One Solution Cell Proliferation Assay (manufactured by Promega Corporation). The amount of the HBs antigen in the collected culture supernatant was measured using AlphaLISA Hepatitis B Virus Surface Antigen (HBsAg) Kit (manufactured by PerkinElmer, Inc.). In addition, the collected culture supernatant was treated with a mixture of Buffer AL (manufactured by Qiagen) and Proteinase K (manufactured by Thermo Fisher Scientific, Inc.), and the DNA of HBV (the HBV DNA) in the obtained solution was quantified by a real-time PCR method. Regarding each of the markers, the relative value (%) in each of the dsRNA-added wells in each concentration to the negative control dsRNA-added well was calculated (Fig. 3 and Fig. 4).

Due to the knockdown of RBFOX1, SRSF10, ELAVL1, and PUM2, the HBs antigen reduced by up to 30%, and due to the knockdown of SRSF9, SNRPA, YTHDC1, and SRSF1, the HBs antigen reduced by up to 70% to 90% (Fig. 3). In addition, due to the knockdown of PUM2 and SRSF1, the HBV DNA reduced by up to 40 to 50%, and due to the knockdown of YTHDC1, the HBV DNA reduced by up to 80%. On the other hand, the knockdown of these genes did not affect cell viability (Fig. 4). From the above, it has been revealed that in a case where a protein that binds to the Enh I-equivalent region and the Enh II-equivalent region on the RNA encoding the HBs antigen, anti-HBV effects such as inhibition of HBV protein production and reduction of HBV DNA expression level can be obtained.

### [Test Example 4]

### (Assay using Enh I/Enh II region-deficient construct and genotype study)

PCR reaction was carried out in a reaction solution containing 1× PrimeSTAR Max DNA Polymerase (manufactured by Takara Bio Inc.) and a 0.2 µmol/L primer. PCR was carried out using the HBV genome DNA (GenBank: AF462041.1; hereinafter also referred to as the "genotype A") as a template and using a primer combination of a primer consisting of the nucleotide sequence of SEQ ID NO: 13 and a primer consisting of the nucleotide sequence of SEQ ID NO: 14, under the condition of 35 cycles at 98°C for 10 seconds, 58°C for 5 seconds, and 72°C for 10 seconds, to obtain an amplified fragment of the region of 1,086 bp containing the 3'UTR sequence (hereinafter, also referred to as "3'UTR (genotype A)") of the gene encoding the HBs antigen, in which restriction enzyme sites of Xba I and Sal I were introduced. This fragment was treated with a combination of Xba I (manufactured by Takara Bio Inc.) and Sal I (manufactured by Takara Bio Inc.) and inserted into the Xba I and Sal I sites of PreS2/S-Luc-3'UTR (genotype C) prepared in Test Example 1 to obtain a ligated plasmid. Hereinafter, this plasmid is also referred to as "PreS2/S-Luc-3'UTR (genotype A)". PCR was carried out using the PreS2/S-Luc-3'UTR (genotype A) plasmid as a template and using a primer combination of a primer consisting of the nucleotide sequence of SEQ ID NO: 15 and a primer consisting of the nucleotide sequence of SEQ ID NO: 10 and a primer combination of a primer consisting of the nucleotide sequence of SEQ ID NO: 7 and a primer consisting of the nucleotide sequence of SEQ ID NO: 16, under the condition of 35 cycles at 98°C for 10 seconds, 58°C for 5 seconds, and 72°C for 30 seconds, to obtain amplified fragments for constructing an Enh I region-deficient plasmid. These fragments were ligated using an In-Fusion HD Cloning Kit (manufactured by Takara Bio Inc.) to obtain plasmids lacking the Enh I region in the PreS2/S-Luc-3'UTR (genotype A) plasmid. Hereinafter, this plasmid is also referred to as "PreS2/S-Luc-3'UTR (genotype A) dEnh I". PCR was carried out using the PreS2/S-Luc-3'UTR (genotype A) plasmid as a template and using a primer combination of a primer consisting of the nucleotide sequence of SEQ ID NO: 17 and a primer consisting of the nucleotide sequence of SEQ ID NO: 10 and a primer combination of a primer consisting of the nucleotide sequence of SEQ ID NO: 7 and a primer consisting of the nucleotide sequence of SEQ ID NO: 18, under the condition of 35 cycles at 98°C for 10 seconds, 58°C for 5 seconds, and 72°C for 30 seconds, to obtain amplified fragments for constructing an Enh II region-deficient plasmid. These fragments were ligated using an In-Fusion HD Cloning Kit (manufactured by Takara Bio Inc.) to obtain plasmids lacking the Enh II region in the PreS2/S-Luc-3'UTR (genotype A) plasmid. Hereinafter, this plasmid is also referred to as "PreS2/S-Luc-3'UTR (genotype A) dEnh II".

**[Table 3]**

| SEQ ID NO | Sequence (5' → 3') |
|---|---|
| 13 | TAGTTCTAGAACCCTAACAAAACAAAAAGA |
| 14 | TAGTGTCGACTTTATAAGGGTCAATGTCCA |
| 15 | CCTGCCTTAATGCCTCCGATCCATACTGCG |
| 16 | AGGCATTAAGGCAGGATATC |
| 17 | AACGCCCATCAGATCATTAGGAGGCTGTAG |
| 18 | GATCTGATGGGCGTTCACGG |

PCR was carried out using the HBV genome DNA (SEQ ID NO: 19, hereinafter also referred to as the "genotype B") as a template and using a primer combination of a primer consisting of the nucleotide sequence of SEQ ID NO: 20 and a primer consisting of the nucleotide sequence of SEQ ID NO: 21, under the condition of 35 cycles at 98°C for 10 seconds, 58°C for 5 seconds, and 72°C for 10 seconds, to obtain an amplified fragment of the region of 1,095 bp containing the 3'UTR sequence (hereinafter, also referred to as "3'UTR (genotype B)") of the gene encoding the HBs antigen, in which restriction enzyme sites of Xba I and Sal I were introduced. This fragment was treated with a combination of Xba I (manufactured by Takara Bio Inc.) and Sal I (manufactured by Takara Bio Inc.) and inserted into the Xba I and Sal I sites of PreS2/S-Luc-3'UTR (genotype C) prepared in Test Example 1 to obtain a ligated plasmid. Hereinafter, this plasmid is also referred to as "PreS2/S-Luc-3'UTR (genotype B)". PCR was carried out using the PreS2/S-Luc-3'UTR (genotype B) plasmid as a template and using a primer combination of a primer consisting of the nucleotide sequence of SEQ ID NO: 22 and a primer consisting of the nucleotide sequence of SEQ ID NO: 10 and a primer combination of a primer consisting of the nucleotide sequence of SEQ ID NO: 7 and a primer consisting of the nucleotide sequence of SEQ ID NO: 23, under the condition of 35 cycles at 98°C for 10 seconds, 58°C for 5 seconds, and 72°C for 30 seconds, to obtain amplified fragments for constructing an Enh I region-deficient plasmid. These fragments were ligated using an In-Fusion HD Cloning Kit (manufactured by Takara Bio Inc.) to obtain plasmids lacking the Enh I region in the PreS2/S-Luc-3'UTR (genotype B) plasmid. Hereinafter, this plasmid is also referred to as "PreS2/S-Luc-3'UTR (genotype B) dEnh I". PCR was carried out using the PreS2/S-Luc-3'UTR (genotype B) plasmid as a template and using a primer combination of a primer consisting of the nucleotide sequence of SEQ ID NO: 24 and a primer consisting of the nucleotide sequence of SEQ ID NO: 10 and a primer combination of a primer consisting of the nucleotide sequence of SEQ ID NO: 7 and a primer consisting of the nucleotide sequence of SEQ ID NO: 25, under the condition of 35 cycles at 98°C for 10 seconds, 58°C for 5 seconds, and 72°C for 30 seconds, to obtain amplified fragments for constructing an Enh II region-deficient plasmid. These fragments were ligated using an In-Fusion HD Cloning Kit (manufactured by Takara Bio Inc.) to obtain plasmids lacking the Enh II region in the PreS2/S-Luc-3'UTR (genotype B) plasmid. Hereinafter, this plasmid is also referred to as "PreS2/S-Luc-3'UTR (genotype B) dEnh II".

**[Table 4]**

| SEQ ID NO | Sequence (5' → 3') |
|---|---|
| 1 9 | TTTTTCACCTCTGCCTAATCATCTCATGTTCATGTCCTACTGTTCAAGCCTCCAAGCTGTGCCTTGGGTGGCTT TGGGGCATGGACATTGACCCGTATAAAGAATTTGGAGCTTCTGTGGAGTTACTCTCTTTTTTGCCTTCTGACTT CTTTCCTTCTATTCGAGATCTCCTCGACACCGCCTCTGCTCTGTATCGGGAGGCCTTAGAGTCTCCGGAACATT GTTCACCTCACCATACGGCACTCAGGCAAGCTATTCTGTGTTGGGGTGAGTTAATGAATCTAGCCACCTGGGTG GGAAGTAATTTGGAAGATCCAGCATCCAGGGAATTAGTAGTCAGCTATGTCAACGTTAATATGGGCTTAAAAAT CAGACAACTATTGTGGTTTCACATTTCCTGTCTTACTTTTGGGAGAGAAACTGTTCTTGAATATTTGGTGTCTT TTGGAGTGTGGATTCGCACTCCTCCTGCATATAGACCACAAAATGCCCCTATCTTATCAACACTTCCGGAAACT ACTGTTGTTAGACGAAGAGGCAGGTCCCCTAGAAGAAGAACTCCCTCGCCTCGCAGACGAAGGTCTCAATCGCC GCGTCGCAGAAGATCTCAATCTCGGGAATCTCAATGTTAGTATTCCTTGGACACATAAGGTGGGAAACTTTACG GGGCTTTATTCTTCTACGGTACCTTGCTTTAATCCTAAATGGCAAACTCCTTCTTTTCCTGACATTCATTTGCA GGAGGACATTGTTGATAGATGTAAGCAATTTGTGGGGCCCCTTACAGTAAATGAAAACAGGAGACTAAAATTAA TTATGCCTGCTAGGTTTTATCCCAATGTTACTAAATATTTGCCCTTAGATAAAGGGATCAAACCGTATTATCCA GAGTATGTAGTTAATCATTACTTCCAGACGCGACATTATTTACACACTCTTTGGAAGGCGGGGATCTTATATAA AAGAGAGTCCACACGTAGCGCCTCATTTTGCGGGTCACCATATTCTTGGGAACAAGATCTACAGCATGGGAGGT TGGTCTTCCAAACCTCGAAAAGGCATGGGGACAAATCTTTCTGTCCCCAATCCCCTGGGATTCTTCCCCGATCA TCAGTTGGACCCTGCATTCAAAGCCAACTCAGAAAATCCAGATTGGGACCTCAACCCGCACAAGGACAACTGGC CGGACGCCAACAAGGTGGGAGTGGGAGCATTCGGGCCAGGGTTCACCCCTCCCCATGGGGGACTGTTGGGGTGG AGCCCTCAGGCTCAGGGCCTACTCACAACTGTGCCAGCAGCTCCTCCTCCTGCCTCCACCAATCGGCAGTCAGG AAGGCAGCCTACTCCCTTATCTCCACCTCTAAGGGACACTCATCCTCAGGCCATGCAGTGGAACTCCACCACTT TCCACCAAACTCTTCAAGATCCCAGAGTCAGGGCCCTGTACTTCCCTGCTGGTGGCTCCAGTTCAGGAACAGTG AGCCCTGCTCAGAATACTGTCTCTGCCATATCGTCAATCTTATCGAAGACTGGGGACCCTGTACCGAACATGGA GAACATCGCATCAGGACTCCTAGGACCCCTGCTCGTGTTACAGGCGGGGTTTTTCTTGTTGACAAAAATCCTCA CAATACCACAGAGTCTAGACTCGTGGTGGACTTCTCTCAATTTTCTAGGGGGAACACCCGTGTGTCTTGGCCAA AATTCGCAGTCCCAAATCTCCAGTCACTCACCAACCTGTTGTCCTCCAATTTGTCCTGGTTATCGCTGGATGTG TCTGCGGCGTTTTATCATCTTCCTCTGCATCCTGCTGCTATGCCTCATCTTCTTGTTGGTTCTTCTGGACTATC AAGGTATGTTGCCCGTTTGTCCTCTACTTCCAGGATCATCAACGACCAGCACCGGACCATGCAAAACCTGCACA ACTCCTGCTCAAGGAACCTCTATGTTTCCCTCATGTTGCTGTACAAAACCTACGGACGGAAACTGCACCTGTAT TCCCATCCCATCATCTTGGGCTTTCGCAAAATACCTATGGGAGTGGGCCTCAGTCCGTTTCTCTTGGCTCAGTT TACTAGTGCCATTTGTTCAGTGGTTCGTAGGGCTTTCCCCCACTGTCTGGCTTTCAGTTATATGGATGATGTGG TTTTGGGGGCCAAGTCTGTACAACATCTTGAATCCCTTTATGCCGCTGTTACCAATTTTCTTTTGTCTTTGGGT ATACATTTAAACCCTCACAAAACAAAAAGATGGGGATATTCCCTTAACTTCATGGGATATGTAATTGGGAGTTG GGGCACATTGCCACAAGAACATATTGTACAAAAAATCAAAATGTGTTTTAGGAAACTTCCTGTAAACAGGCCTA TTGATTGGAAAGTATGTCAACGAATTGTGGGTCTTTTGGGGTTTGCCGCCCCTTTCACGCAATGTGGATATCCT GCTTTAATGCCTTTATATGCATGTATACAAGCAAAACAGGCTTTTACTTTCTCGCCAACTTACAAGGCCTTTCT AAGTAAACAGTATCTGAACCTTTACCCCGTTGCTCGGCAACGGCCTGGTCTGTGCCAAGTGTTTGCTGATGCAA CCCCCACTGGTTGGGGCTTGGCCATAGGCCATCAGCGCATGCGTGGAACCTTTGTGTCTCCTCTGCCGATCCAT ACTGCGGAACTCCTAGCCGCTTGTTTTGCTCGCAGCAGGTCTGGGGCAAAACTCATCGGGACTGACAATTCTGT CGTGCTCTCCCGCAAGTATACATCATTTCCATGGCTGCTAGGTTGTGCTGCCAACTGGATCCTGCGCGGGACGT CCTTTGTTTACGTCCCGTCGGCGCTGAATCCCGCGGACGACCCCTCCCGGGGCCGCTTGGGGCTCTACCGCCCG CTTCTCCGCCTGTTGTACCGACCGACCACGGGGCGCACCTCTCTTTACGCGGACTCCCCGTCTGTGCCTTCTCA TCTGCCGGACCGTGTGCACTTCGCTTCACCTCTGCACGTCGCATGGAGACCACCGTGAACGCCCACGGGAACCT GCCCAAGGTCTTGCATAAGAGGACTCTTGGACCCAAGGTCTTGCATAAGAGGACTCTTGGACTTTCAGCAATGT CAACGACCGACCTTGAGGCATACTTCAAAGACTGTGTGTTTAACGAGTGGGAGGAGTTGGGGGTTGTACTAGGA GGCTGTAGGCATAAATTGGTGTGTTCACCAGCACCATGCAAC |

**[Table 5]**

| SEQ ID NO | Sequence (5' → 3') |
|---|---|
| 20 | TAGTTCTAGAACCCTCACAAAACAAAAAGA |
| 21 | TAGTGTCGACTTTATACGGGTCAATGTCCA |
| 22 | CCTGCTTTAATGCCTCCGATCCATACTGCG |
| 23 | AGGCATTAAAGCAGGATATC |
| 24 | AAGAGGACTCTTGGAAGGCTGTAGGCATAA |
| 25 | TCCAAGAGTCCTCTTATGCA |

PCR was carried out using the HBV genome DNA (GenBank: U95551.1; hereinafter also referred to as the "genotype D") as a template and using a primer combination of a primer consisting of the nucleotide sequence of SEQ ID NO: 26 and a primer consisting of the nucleotide sequence of SEQ ID NO: 27, under the condition of 35 cycles at 98°C for 10 seconds, 58°C for 5 seconds, and 72°C for 15 seconds, to obtain an amplified fragment of the region of 1,086 bp containing the 3'UTR sequence (hereinafter, also referred to as "3'UTR (genotype D)") of the gene encoding the HBs antigen, in which restriction enzyme sites of Xba I and Sal I were introduced. This fragment was treated with a combination of Xba I (manufactured by Takara Bio Inc.) and Sal I (manufactured by Takara Bio Inc.) and inserted into the Xba I and Sal I sites of PreS2/S-Luc-3'UTR (genotype C) prepared in Test Example 1 to obtain a ligated plasmid. Hereinafter, this plasmid is also referred to as "PreS2/S-Luc-3'UTR (genotype D)". PCR was carried out using the PreS2/S-Luc-3'UTR (genotype D) plasmid as a template and using a primer combination of a primer consisting of the nucleotide sequence of SEQ ID NO: 28 and a primer consisting of the nucleotide sequence of SEQ ID NO: 10 and a primer combination of a primer consisting of the nucleotide sequence of SEQ ID NO: 7 and a primer consisting of the nucleotide sequence of SEQ ID NO: 29, under the condition of 35 cycles at 98°C for 10 seconds, 58°C for 5 seconds, and 72°C for 30 seconds, to obtain amplified fragments for constructing an Enh I region-deficient plasmid. These fragments were ligated using an In-Fusion HD Cloning Kit (manufactured by Takara Bio Inc.) to obtain plasmids lacking the Enh I region in the PreS2/S-Luc-3'UTR (genotype D) plasmid. Hereinafter, this plasmid is also referred to as "PreS2/S-Luc-3'UTR (genotype D) dEnh I". PCR was carried out using the PreS2/S-Luc-3'UTR (genotype D) plasmid as a template and using a primer combination of a primer consisting of the nucleotide sequence of SEQ ID NO: 30 and a primer consisting of the nucleotide sequence of SEQ ID NO: 10 and a primer combination of a primer consisting of the nucleotide sequence of SEQ ID NO: 7 and a primer consisting of the nucleotide sequence of SEQ ID NO: 31, under the condition of 35 cycles at 98°C for 10 seconds, 58°C for 5 seconds, and 72°C for 30 seconds, to obtain amplified fragments for constructing an Enh II region-deficient plasmid. These fragments were ligated using an In-Fusion HD Cloning Kit (manufactured by Takara Bio Inc.) to obtain plasmids lacking the Enh II region in the PreS2/S-Luc-3'UTR (genotype D) plasmid. Hereinafter, this plasmid is also referred to as "PreS2/S-Luc-3'UTR (genotype D) dEnh II".

**[Table 6]**

| SEQ ID NO | Sequence (5'→3') |
|---|---|
| 26 | TAGTTCTAGAACCCTAACAAAACAAAGAGA |
| 27 | TAGTGTCGACTTTATAAGGGTCGATGTCCA |
| 28 | CCTGCGTTAATGCCCCCGATCCATACTGCG |
| 29 | GGGCATTAACGCAGGATAAC |
| 30 | ACGCCCACCGAATGTACTAGGAGGCTGTAG |
| 31 | ACATTCGGTGGGCGTTCACG |

A reporter assay was carried out using HepG 2.2.15 cells according to the following procedure.

The following medium was used as a cell culture medium.

DMEM/F-12, GlutaMAX (manufactured by Thermo Fisher Scientific, Inc.) + 5 µg/mL insulin (manufactured by FUJIFILM Wako Pure Chemical Corporation) + 1% penicillin/streptomycin (manufactured by FUJIFILM Wako Pure Chemical Corporation) + 10 mmol/L HEPES (manufactured by FUJIFILM Wako Pure Chemical Corporation) + 50 µmol/L hydrocortisone (manufactured by FUJIFILM Wako Pure Chemical Corporation) +10 % FBS (manufactured by Equitech-Bio Inc.)

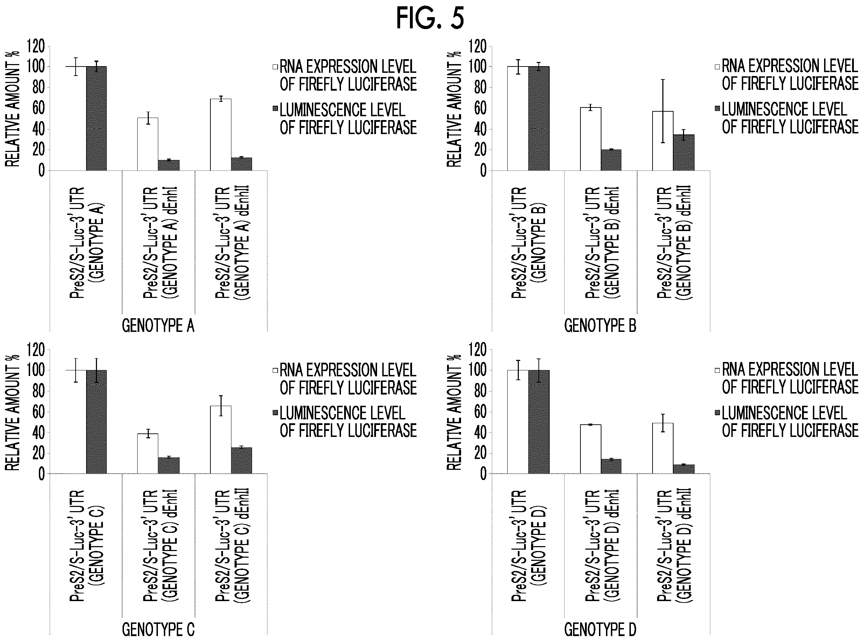

HepG 2.2.15 cells were suspended in the above medium, seeded on a 96-well microtiter plate so that the cell concentration was 1 × 10⁴ cells/100 µL per well, and incubated overnight in a 5% CO₂ incubator at 37°C. 64 ng of any one of various plasmids (PreS2/S-Luc-3'UTR (genotype A), PreS2/S-Luc-3'UTR (genotype A) dEnh I, PreS2/S-Luc-3'UTR (genotype A) dEnh II, PreS2/S-Luc-3'UTR (genotype B), PreS2/S-Luc-3'UTR (genotype B) dEnh I, PreS2/S-Luc-3'UTR (genotype B) dEnh II, PreS2/S-Luc-3'UTR (genotype C), PreS2/S-Luc-3'UTR (genotype C) dEnh I, PreS2/S-Luc-3'UTR (genotype C) dEnh II, PreS2/S-Luc-3'UTR (genotype D), PreS2/S-Luc-3'UTR (genotype D) dEnh I, and PreS2/S-Luc-3'UTR (genotype D) dEnh II), 0.25 ng of a pRL-SV40 plasmid (manufactured by Promega Corporation), and 0.24 µL of TransIT-X2 Transfection Reagent (manufactured by Mirus Bio LLC) were diluted with 7.8 µL of serum-free medium (Thermo Fisher Scientific, Inc.) and incubated at room temperature for 20 minutes. 8 µL of this plasmid-TransIT-X2 Transfection Reagent mixture was added to the wells seeded with HepG 2.2.15 cells described above and further incubated for one day. Next, the medium was exchanged at 100 µL/well with the above-described cell culture medium, and incubation was further carried out at 37°C for 24 hours in a 5% CO₂ incubator. After incubation, the luminescence by firefly luciferase and the luminescence by Renilla luciferase were detected in Dual-Luciferase Reporter Assay System (manufactured by Promega Corporation). Regarding each of the wells, the luminescence level of firefly luciferase corrected by the luminescence level of Renilla luciferase was calculated. Then, the results of calculating the relative value (%) of the luminescence level of luciferase in the genotype-defective plasmid-added wells to the luminescence level of firefly luciferase in the respective PreS2/S-Luc-3'UTR (genotype A), PreS2/S-Luc-3'UTR (genotype B), PreS2/S-Luc-3'UTR (genotype C), and PreS2/S-Luc-3'UTR (genotype D) plasmid-added wells are shown in Fig. 5.

HepG 2.2.15 cells were suspended in the above medium, seeded on a 24-well microtiter plate so that the cell concentration was 1 × 10⁵ cells/500 µL per well, and incubated overnight in a 5% CO₂ incubator at 37°C. 500 ng of any one of various plasmids (PreS2/S-Luc-3'UTR (genotype A), PreS2/S-Luc-3'UTR (genotype A) dEnh I, PreS2/S-Luc-3'UTR (genotype A) dEnh II, PreS2/S-Luc-3'UTR (genotype B), PreS2/S-Luc-3'UTR (genotype B) dEnh I, PreS2/S-Luc-3'UTR (genotype B) dEnh II, PreS2/S-Luc-3'UTR (genotype C), PreS2/S-Luc-3'UTR (genotype C) dEnh I, PreS2/S-Luc-3'UTR (genotype C) dEnh II, PreS2/S-Luc-3'UTR (genotype D), PreS2/S-Luc-3'UTR (genotype D) dEnh I, and PreS2/S-Luc-3'UTR (genotype D) dEnh II), and 1.5 µL of TransIT-X2 Transfection Reagent (manufactured by Mirus Bio LLC) were diluted with 50 µL of serum-free medium (Thermo Fisher Scientific, Inc.) and incubated at room temperature for 20 minutes. 52 µL of this plasmid-TransIT-X2 Transfection Reagent mixture was added to the wells seeded with HepG 2.2.15 cells described above and incubated for one day. Next, the medium was exchanged at 500 µL/well with the above-described cell culture medium, and incubation was further carried out at 37°C for one day in a 5% CO₂ incubator. Then, the culture supernatant was discarded, the cells were washed with phosphate buffered saline (1,000 µL/well, manufactured by FUJIFILM Wako Pure Chemical Corporation), and total RNA was extracted using an RNeasy Mini Kit (manufactured by Qiagen). The extracted total RNA was reverse transcribed using a PrimeScript RT Reagent Kit with gDNA Eraser (manufactured by Takara Bio Inc.) to prepare cDNA. The expression level of the firefly luciferase RNA was quantified based on the prepared cDNA, and the results of calculating the relative value (%) of the expression level of luciferase RNA in the genotype-defective plasmid-added wells to the expression level of firefly luciferase RNA in the respective PreS2/S-Luc-3'UTR (genotype A), PreS2/S-Luc-3'UTR (genotype B), PreS2/S-Luc-3'UTR (genotype C), and PreS2/S-Luc-3'UTR (genotype D) plasmid-added wells are shown in Fig. 5.

In the genotype A, the relative luminescence level derived from the firefly luciferase protein was reduced by about 87% to 90% due to the deletion of the Enh I region or the Enh II region (Fig. 5). On the other hand, the relative expression level of the firefly luciferase RNA decreased by 31% to 49% due to the deletion of the Enh I region or the Enh II region.

In the genotype B, the relative luminescence level derived from the firefly luciferase protein was reduced by about 65% to 80% due to the deletion of the Enh I region or the Enh II region. On the other hand, the relative expression level of the firefly luciferase RNA decreased by 39% to 43% due to the deletion of the Enh I region or the Enh II region.

In the genotype C, the relative luminescence level derived from the firefly luciferase protein was reduced by about 74% to 84% due to the deletion of the Enh I region or the Enh II region. On the other hand, the relative expression level of the firefly luciferase RNA decreased by 34% to 61% due to the deletion of the Enh I region or the Enh II region.

In the genotype D, the relative luminescence level derived from the firefly luciferase protein was reduced by about 86% to 91% due to the deletion of the Enh I region or the Enh II region. On the other hand, the relative expression level of the firefly luciferase RNA decreased by 51% to 52% due to the deletion of the Enh I region or the Enh II region.

All of the above results indicate that the Enh I region and the Enh II region contribute to the process of translation from RNA to protein. That is, it has been revealed that the region corresponding to Enh I or the region corresponding to Enh II, on the HBV genome DNA, functions on the RNA and controls the expression level of the protein, which is common to a plurality of genotypes of HBV.

The pharmaceutical composition according to the embodiment of the present invention exhibits an excellent anti-HBV activity and is useful as an anti-hepatitis B virus agent.

## Claims

1. A pharmaceutical composition that inhibits a production of a hepatitis B virus protein,
wherein the pharmaceutical composition inhibits an expression or a function of an RNA-binding protein that binds to at least one sequence selected from a hepatitis B virus RNA sequence corresponding to an enhancer I region sequence on a hepatitis B virus genome DNA or a hepatitis B virus RNA sequence corresponding to an enhancer II region sequence on the hepatitis B virus genome DNA.

2. The pharmaceutical composition according to claim 1,
wherein the hepatitis B virus RNA sequence corresponding to the enhancer I region sequence on the hepatitis B virus genome DNA is a sequence having 80% or more of a sequence identity with SEQ ID NO: 1, and
the hepatitis B virus RNA sequence corresponding to the enhancer II region sequence on the hepatitis B virus genome DNA is a sequence having 80% or more of a sequence identity with SEQ ID NO: 2.

3. The pharmaceutical composition according to claim 1 or 2,
wherein the hepatitis B virus RNA sequence corresponding to the enhancer I region sequence on the hepatitis B virus genome DNA is a sequence having 90% or more of a sequence identity with SEQ ID NO: 1, and
the hepatitis B virus RNA sequence corresponding to the enhancer II region sequence on the hepatitis B virus genome DNA is a sequence having 90% or more of a sequence identity with SEQ ID NO: 2.

4. The pharmaceutical composition according to any one of claims 1 to 3,
wherein the hepatitis B virus protein is an HBs antigen protein.

5. The pharmaceutical composition according to any one of claims 1 to 4,
wherein the pharmaceutical composition reduces an expression level of the hepatitis B virus genome DNA.

6. The pharmaceutical composition according to any one of claims 1 to 5,
wherein the pharmaceutical composition reduces an expression level of a completely closed double-stranded DNA.

7. The pharmaceutical composition according to any one of claims 1 to 6,
wherein the RNA-binding protein is at least one protein selected from RBFOX1, ELAVL2, MBNL1, RBMX, SRSF9, SRSF10, SNRPA, ELAVL1, PUM2, YTHDC1, SRSF1, KHDRBS3, or EIF4B.

8. A screening method which is a method of screening a substance that inhibits a production of a hepatitis B virus protein, the method comprising
identifying a substance that inhibits an expression or a function of an RNA-binding protein that binds to at least one sequence selected from a hepatitis B virus RNA sequence corresponding to an enhancer I region sequence on a hepatitis B virus genome DNA or a hepatitis B virus RNA sequence corresponding to an enhancer II region sequence on the hepatitis B virus genome DNA.

9. The screening method according to claim 8,
wherein the hepatitis B virus RNA sequence corresponding to the enhancer I region sequence on the hepatitis B virus genome DNA is a sequence having 80% or more of a sequence identity with SEQ ID NO: 1, and
the hepatitis B virus RNA sequence corresponding to the enhancer II region sequence on the hepatitis B virus genome DNA is a sequence having 80% or more of a sequence identity with SEQ ID NO: 2.

10. The screening method according to claim 8 or 9,
wherein the hepatitis B virus protein is an HBs antigen protein.

11. A method of screening a substance that inhibits a production of a hepatitis B virus protein, the method comprising:
(a) a step of identifying an RNA-binding protein that binds to at least one sequence selected from a hepatitis B virus RNA sequence corresponding to an enhancer I region sequence on a hepatitis B virus genome DNA or a hepatitis B virus RNA sequence corresponding to an enhancer II region sequence on the hepatitis B virus genome DNA;
(b) a step of bringing cells infected with a hepatitis B virus or cells expressing a hepatitis B virus into contact with a test substance that inhibits a function or an activity of an identified RNA-binding protein or with a test substance that reduces an expression level of the protein;
(c) a step of measuring an ability of producing a hepatitis B virus protein in the cells infected with a hepatitis B virus or the cells expressing a hepatitis B virus; and
(d) a step of selecting a substance having an activity of inhibiting a production of the hepatitis B virus protein of the step (c).

12. The method according to claim 11,
wherein the (a) includes a step of using an RNA-binding protein sequence database.

13. The method according to claim 11,
wherein the test substance of the step (b) is an antibody, a peptide, a protein, a non-peptide compound, a synthetic compound, an antisense nucleic acid, a double-stranded RNA, an adeno-associated virus vector, or a CRISPR-Cas vector system.

14. The method according to claim 11,
wherein the (c) includes a step of measuring a hepatitis B virus protein in a culture supernatant of the cells infected with hepatitis B virus or the cells expressing hepatitis B virus.

15. The method according to claim 11,
wherein the substance of the step (d) is an antibody, a peptide, a protein, a non-peptide compound, a synthetic compound, an antisense nucleic acid, a double-stranded RNA, an adeno-associated virus vector, or a CRISPR-Cas vector system.
